# EUROPEAN PATENT SPECIFICATION

(11) **EP 4 175 559 B1**
(45) Date of publication and mention of the grant of the patent: **03.09.2025**
(21) Application number: 21831961.4
(22) Date of filing: 01.07.2021
(51) Int. Cl.: A61B 10/02

(54) **TISSUE PENETRATOR**
GEWEBEPENETRATOR
PÉNÉTRATEUR DE TISSU

(30) Priority: 01.07.2020 US 202063046711 P
(43) Date of publication of application: 10.05.2023
(73) Proprietor: Limaca Medical Ltd., 1925000 Ein HaEmek (IL)
(72) Inventor: KLEIN, Assaf, 3885700 Kibbutz HaMaApil (IL)
(74) Representative: Nederlandsch Octrooibureau
(86) International application number: PCT/IL2021/050808
(87) International publication number: WO 2022/003691

(56) References cited:
- WO-A1-2013/168166
- WO-A1-2013/168166
- WO-A1-2016/010735
- US-A1- 2015 272 556
- US-A1- 2015 272 556
- US-A1- 2018 140 289
- US-A1- 2018 140 289
- US-B2- 10 441 254
- US-B2- 10 441 254

## Description

### RELATED APPLICATION

This application claims the benefit of priority of U.S. Provisional Patent Application No. 63/046,711 filed on 1 July 2020.

### FIELD AND BACKGROUND OF THE INVENTION

The present invention, in some embodiments thereof, relates to a tissue penetrator and, more particularly, but not exclusively, to a tissue penetrator of a biopsy device.

Additional background includes U.S. Patent application No. US20180140289A1 disclosing a biopsy device as defined in the preamble of claim 1. The biopsy device may include a first jaw having a first distal tip configured to pierce tissue, and a second jaw movable relative to the first jaw between a closed configuration where the first jaw and the second jaw are axially aligned, and an open configuration where the first jaw and the second jaw are offset from one another, the second jaw having a second distal tip proximal to the first distal tip in the closed configuration.

U.S. Patent No. US10441254B2 disclosing an apparatus, comprising: a biopsy needle having a distal end and a proximal end, all or a portion of the biopsy needle being movable between a collapsed state and an expanded state; a sheath having a distal end and a proximal end, wherein the biopsy needle in the collapsed state is contained within the sheath, and in the expanded state at least the distal end of the biopsy needle extends from the distal end of the sheath; wherein the biopsy needle is provided with two or more sleeves, which at least partially overlap in the collapsed configuration and which cylindrically expand to provide an increased radius, relative to a radius in the collapsed state, for sampling in the expanded configuration, and wherein a cross section of the distal end of the biopsy needle in the expanded state is a substantially circular cross section.

U.S. Patent application No. US20150272556A1 disclosing a biopsy assembly including a biopsy catheter having a proximal portion and a distal portion, a navigation catheter configured to receive the biopsy catheter for positioning the biopsy catheter adjacent target tissue, wherein the biopsy catheter is configured to be received within the navigation catheter, a coring component, and an anchoring component configured to anchor the biopsy catheter to target tissue. The coring component includes a proximal region and a distal region, the distal region formed of one or more distally extending blades and the proximal region being coupled to the distal portion of the biopsy catheter and the one or more blades are configured to penetrate target tissue and sever a tissue sample from the target tissue.

International Patent publication No. WO2013168166A1 disclosing a tissue sampling apparatus and method are disclosed. Some embodiments comprise a biopsy needle having a laterally expandable distal portion comprising spines which move away from each other when unconstrained and which move towards each other when constrained to do so by a sheath. In some embodiments said spines comprise inward-pointing teeth designed to catch tissue samples when said needle retracted from tissue. A method of use comprises providing such a needle within a sheath, advancing the sheath to near a sampling site, advancing needle beyond sheath to expand the expandable portion, retracting needle into sheath so that the expandable portion collapses and tissue samples are trapped between the teeth and spines, and removing the needle and the samples it contains from the body.

International Patent publication No. WO2016010735A1 disclosing a device for collecting a tissue sample includes an outer sheath extending longitudinally from a proximal end to a distal end and including a lumen extending therethrough. The device also includes a tissue collecting member movably housed within the lumen and biased toward an expanded configuration in which the member is curved about a longitudinal axis thereof and longitudinal edges thereof are separated from one another. The member is movable between a constrained position, in which the tissue collecting is received within the lumen and constrained by a surface thereof such that longitudinal edges of the member are drawn toward one another to define a channel therein, and a tissue collecting position, in which the member moves distally past the distal end of the outer sheath to revert to the biased expanded configuration to cut a target tissue into which it is inserted.

### SUMMARY OF THE INVENTION

The invention relates to a biopsy assembly as defined in claim 1. Additional embodiments are defined in the dependent claims.

Unless otherwise defined, all technical and/or scientific terms used herein have the same meaning as commonly understood by one of ordinary skill in the art to which the invention pertains. Although methods and materials similar or equivalent to those described herein can be used in the practice or testing of embodiments of the invention, exemplary methods and/or materials are described below. In case of conflict, the patent specification, including definitions, will control. In addition, the materials, methods, and examples are illustrative only and are not intended to be necessarily limiting.

### BRIEF DESCRIPTION OF THE SEVERAL VIEWS OF THE DRAWING(S)

Some embodiments of the invention are herein described, by way of example only, with reference to the accompanying drawings. With specific reference now to the drawings in detail, it is stressed that the particulars shown are by way of example and for purposes of illustrative discussion of embodiments of the invention. In this regard, the description taken with the drawings makes apparent to those skilled in the art how embodiments of the invention may be practiced.

In the drawings:
Fig. 1 is a flow chart of a general process for penetrating through tissue and collapsing a tissue penetrator, according to some exemplary embodiments of the invention;
Fig. 2A is a state diagram scheme showing changes in tissue penetrator between an expanded state and a collapsed state, according to some exemplary embodiments of the invention
Figs. 2B and 2C are a schematic illustrations of a straight cut (2B) and penetration of a tube, for example a biopsy tube through a straight cut (2C), according to some exemplary embodiments of the invention;
Fig. 2D is a schematic illustration showing penetration of a tube, for example a biopsy tube through a curved, for example an arc-shaped cut, according to some exemplary embodiments of the invention;
Fig. 3 is a flow chart of a general process for advancing a tissue penetrator through tissue, and collapsing and removing the tissue penetrator to allow, for example, tissue sampling, according to some exemplary embodiments of the invention;
Figs. 4A and 4B are block diagrams of a tissue penetrator having a collapsible cutting portion, according to some exemplary embodiments of the invention;
FIGs. 5A and 5B are schematic illustrations showing reversibly coupling between a tissue penetrator and a biopsy tube, according to some exemplary embodiments of the invention;
Figs. 6A-6E are schematic illustrations showing penetration through tissue, retraction of a tissue penetrator and tissue sampling, according to some exemplary embodiments of the invention;
Figs. 7A-7H are schematic illustrations of different types of tissue penetrators with different collapsible tissue penetrating portions, for example cutting portions, according to some exemplary embodiments of the invention;
Figs. 8A-8E are schematic illustrations of a tissue penetrator having a shaft and a collapsible tissue penetrating portion, for example a collapsible cutting portion, connected to the shaft in an expanded state, according to some exemplary embodiments of the invention;
FIGs. 9A and 9B are schematic illustrations showing the tissue penetrator of Figs. 8A-8E in a collapsed state, according to some exemplary embodiments of the invention;
Figs. 10A and 10B are schematic cross-section illustrations of a collapsible tissue penetrating portion in an expanded state (Fig. 10A), and in a collapsed state where the collapsible cutting portion is bended as an arc within a sampling cannula, according to some exemplary embodiments of the invention;
Figs. 10C and 10D are schematic cross -section illustrations of a collapsible tissue penetrating portion, for example a collapsible cutting portion, in an expanded state (Fig. 10D) and in a collapsed state where the collapsible portion is furled, for example rolled, according to some exemplary embodiments of the invention; and
Figs. 11A and 11B are schematic illustrations showing a tissue penetrator with an integral collapsible tissue penetrating portion, for example a collapsible cutting portion, according to some exemplary embodiments of the invention.

### DESCRIPTION OF SPECIFIC EMBODIMENTS OF THE INVENTION

The present disclosure relates to a tissue penetrator and, more particularly, but not exclusively, to a tissue penetrator of a biopsy device. Throughout the description, the term "embodiment" is to be understood as "example of the disclosure". The invention is solely defined by the claims.

An aspect of some embodiments relates to collapsing at least a portion of a tissue penetrator, for example a stylet, prior to taking biopsy. In some embodiments, a cutting portion of the tissue penetrator is collapsed into a sampling tube, for example a sampling needle, prior to taking biopsy. As used herein, a sampling tube refers to a biopsy tube, and a sampling needle refers to a biopsy needle. In some embodiments, the cutting portion is collapsed into an inner lumen of the sampling tube, for example to allow removal of the tissue penetrator from the body.

According to some embodiments, the cutting portion is collapsed into the sampling tube, for example, when the sampling tube reaches a desired target tissue in the body that needs to be sampled. In some embodiments, the cutting portion is collapsed when the sampling tube is close, for example at a distance smaller than 2 mm, for example a distance smaller than 1 mm, a distance smaller than 0.5mm or any intermediate, smaller or larger values from a desired target tissue. Alternatively, the cutting portion is collapsed when the sampling tube is in contact with a desired target tissue.

According to some embodiments, in an expanded state, the cutting portion is wider than an opening of the sampling tube. In some embodiments, a width of the cutting portion in a collapsed state is smaller than a minimal width, for example a minimal inner diameter, of the sampling tube. In some embodiments, in an expanded state the width of the cutting portion is similar or smaller than an outer diameter or the maximal width of the sampling tube. In some embodiments, in the expanded state, the maximal width of the cutting portion, for example a maximal distance between two points on the cutting portion, is in a range between half of a sampling tube circumference and a diameter of the sampling tube.

According to some embodiments, the cutting portion, for example a collapsible cutting portion, is rigid in an axial direction, for example to allow penetration through a tissue wall or a membrane without collapsing. Additionally, the cutting portion is bendable in a lateral and/or a tangential direction, for example to allow collapsing, for example bending into the tube, for example a biopsy tube. In some embodiments the tissue wall comprises the gastric wall, duodenum wall, the intestine wall, Trachea wall, Bronchial airways walls, blood vessel walls, GI tract walls, esophagus wall, an epithelium layer, the skin, the diaphragm, a pleura, heart wall, and/or heart septum.

According to some embodiments, in an expanded state, the cutting portion is straight, for example when penetrating through a tissue wall with lower self-sealing properties, for example an intestine wall or a duodenum wall, and therefore a straight cut is formed to minimize leakage through the formed cut. Alternatively, in an expanded state, the cutting portion is curved, for example when penetrating through a tissue wall with high self-sealing properties, for example a wall of the stomach, and therefore a curved cut is formed to maximize ease of penetration through the tissue wall. Optionally, the tissue wall is a thick tissue wall having a thickness larger than 1mm, for example larger than 2mm, larger than 3mm or any intermediate, smaller or larger value.

A potential advantage of generating a cut in the tissue which has a maximal width, for example a maximal distance between two points on the cut, which is in a range between half of a circumference length of the sampling tube and a sampling tube diameter may be, to allow easier penetration of the sampling tube through a wall, for example a wall of the stomach, an intestine wall, and/or a duodenum wall, while preventing leakage though the formed cut.

An aspect of some embodiments relates to removing a wide tissue penetrator from a target tissue through a narrow opening of a biopsy tube lumen. In some embodiments, at least a portion of the wide tissue penetrator, for example a cutting portion, is reshaped to enter through the opening. In some embodiments, at least a portion of the tissue penetrator is collapsed to a width smaller than a minimal width, for example inner diameter of the biopsy tube opening. Optionally, the at least a portion of the tissue penetrator is reversibly collapsed to enter through the biopsy tube lumen opening. In some embodiments, at least a portion of the tissue penetrator is collapsed into the biopsy tube lumen without sampling tissue.

According to some embodiments, at least a portion of the tissue penetrator is folded to pass through the opening into the biopsy tube lumen. In some embodiments, the at least a portion of the tissue penetrator is at least partly furled or at least partly rolled to acquire a shape having a cross section which is narrower than the biopsy tube lumen opening. In some embodiments, when the cutting portion is furled, two or more ends of the cutting portion, overlap.

According to some embodiments, the cutting portion of the tissue penetrator is reshaped, by application of external force on the tissue penetrator, for example by retracting the tissue penetrator, for example through the biopsy tube lumen. Alternatively, the cutting portion is reshaped by rotating the tissue penetrator at least 30° degrees, for example at least 90° degrees, 180° degrees, 360° degrees or any intermediate, smaller or larger rotation degree. In some embodiments, the cutting portion is reshaped by a force applied on the cutting portion by biopsy tube body, causing the cutting portion to reshape.

According to some embodiments, the tissue penetrator is used in a biopsy sampling process of a tissue, for example a tissue suspected to be a malignant tissue.

According to some embodiments, an endoscope is navigated towards a target tissue that needs to be sampled. In some embodiments, when reaching a tissue wall, for example a tissue wall surrounding the target tissue, a tissue penetrator is expanded through a distal opening of the endoscope. In some embodiments, expansion of a tissue penetrator comprises expansion of the cutting portion of the tissue penetrator.

According to some embodiments, when reaching a tissue wall of the desired target or any tissue wall in a way to a desired target tissue, the tissue penetrator is advanced distally to the endoscope and through the tissue wall. In some embodiments, the cutting portion of the tissue penetrator forms a cut in the tissue wall, for example by advancing through the tissue wall. In some embodiments, the cutting portion of the tissue penetrator advances up to 5 cm, up to 3 cm, for example up to 1 cm, for example up to 0.5 cm, or any intermediate, smaller or larger distance from the tissue wall cut.

According to some embodiments, the tissue penetrator is part of a biopsy assembly comprising a biopsy tube. In some embodiments, when the endoscope reaches a tissue wall, the biopsy assembly, having an expanded cutting portion extended from a distal end of the biopsy tube, is advanced through the tissue wall. In some embodiments, when the biopsy assembly penetrates through the tissue wall and optionally at least partly into a target tissue, the tissue penetrator blocks an inner lumen of the biopsy tube, preventing penetration of tissue into the biopsy tube.

According to some embodiments, after forming a cut in the tissue wall, the tissue penetrator is retracted into the biopsy tube inner lumen, for example to unblock an inner lumen of the biopsy tube used for tissue sampling. In some embodiments, the biopsy tube is then advanced into the target tissue, to sample the target tissue. In some embodiments, the biopsy tube is advanced while rotating, into the target tissue, for example as described in WO2019155472A1. In some embodiments, the tissue sampling comprises advancement of the biopsy tube one or more times, for example 2, 3, 4 times or any number of times, into the target tissue.

According to some embodiments, the advancement, for example degree of advancement, of at least one of the tissue penetrator, the biopsy tube and/or the biopsy assembly, is controlled by a handle or a control unit of a biopsy device, for example as described in WO2019155472A1.

According to some embodiments, the tissue wall comprises a muscle or any type of tissue located between a biopsy tube, or any type of a tube inserted into the body, and a desired target tissue. A potential advantage of the tissue penetrator may be to allow penetration through a tissue wall without or with minimal sampling of the tissue wall, for example sampling of less than 10%, less than 5%, less than 1% of the volume of the tissue wall compared to the volume of the sampled target tissue.

Although the examples provided in this application describe using a tissue penetrator with a biopsy tube, it should be understood that the tissue penetrator can be used with any tube inserted into the body, for example a drainage tube, a feeding tube, a guiding tube, an insertion tube, a drainage, a dilator, a catheter, injection needle or any type of needle not used for injections, for example an aspiration needle.

Before explaining at least one embodiment of the invention in detail, it is to be understood that the invention is not necessarily limited in its application to the details of construction and the arrangement of the components and/or methods set forth in the following description and/or illustrated in the drawings and/or the Examples. The invention is capable of other embodiments or of being practiced or carried out in various ways.

### Exemplary tissue cutting process

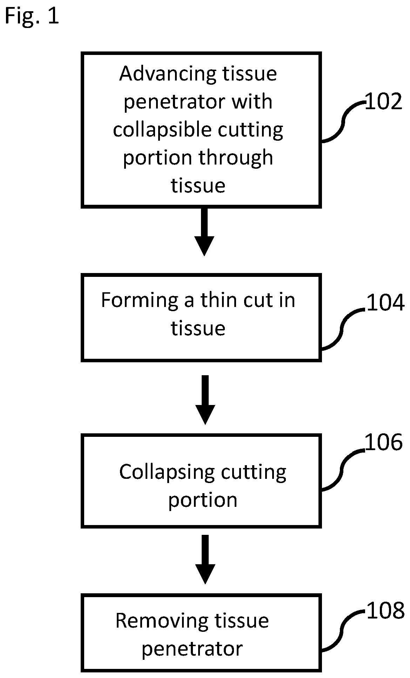

According to some exemplary embodiments, during a biopsy sampling process, a biopsy tube, for example a biopsy needle having an external distal cutting portion used to penetrate through a tissue wall, for example a septum, towards a desired target tissue that needs to be sampled. In some embodiments, in order to allow easy penetration of the sampling tube into the target tissue, a cut in a tissue wall located between the target tissue to be sampled and the biopsy tube is formed by the tissue penetrator. In some embodiments, the cut has a width which is at least as wide as the external width of the biopsy needle and smaller than a half of an outer circumference of the biopsy tube. Reference is now made to fig. 1, describing a process for tissue cutting by a collapsible cutting portion, according to some exemplary embodiments of the invention.

According to some exemplary embodiments, a tissue penetrator having a collapsible cutting portion is advanced through tissue at block 102. In some embodiments, the tissue penetrator is coupled to a biopsy tube, for example a biopsy needle. In some embodiments, at least part of the tissue penetrator is positioned within an inner lumen of the biopsy tube while the collapsible cutting portion of the tissue penetrator extends out from the biopsy tube. Optionally, the collapsible cutting portion extends through a distal opening of the biopsy tube, for example an opening facing the tissue.

According to some exemplary embodiments, the tissue penetrator is fixed to the biopsy tube, for example when advancing through the tissue at block 102. In some embodiments, the tissue penetrator is fixed to the biopsy tube in an axial direction. Additionally, the biopsy tube, for example a rotating biopsy needle, rotates relatively to the tissue penetrator. Alternatively, the tissue penetrator rotates together with the biopsy tube.

According to some exemplary embodiments, the tissue penetrator forms a thin cut through tissue at block 104. In some embodiments, the thin cut is formed by the collapsible cutting portion of the tissue penetrator, for example during the advancement of the tissue penetrator at 102. In some embodiments, the cut formed by the collapsible cutting portion has a width which is similar or smaller than a maximal width of the biopsy tube. In some embodiments, the cut formed by the collapsible cutting portion has a length which is similar or smaller than a maximal width of the biopsy tube. In some embodiments, the cut formed by the collapsible cutting portion has a length which is similar or smaller than half or a circumference length of the biopsy tube.

According to some exemplary embodiments, the thin cut is formed in a wall of a tissue, for example in a wall of the stomach, the wall of the duodenum, the wall of the intestines and/or the wall of the bronchial airways. In some embodiments, the tissue wall is elastic. In some embodiments, the thin cut formed in the tissue wall allows, for example, penetration of a biopsy tube with minimal application of force on the tissue, while preventing or limiting leakage of fluid through the formed thin cut. Optionally, the thin cut allows, for example, an improved self-sealing of the tissue wall after the removal of the biopsy tube from the body.

According to some exemplary embodiments, the collapsible cutting portion in an expanded state, is straight, for example planar. In some embodiments, the straight collapsible cutting portion is used to form a straight cut, for example when the tissue penetrator penetrates through a tissue wall that has limited self-sealing properties, for example the duodenum wall, the intestine wall, Trachea wall, Bronchial airways walls, blood vessel walls, GI tract walls, esophagus wall or any wall of anatomical tubes. Alternatively, the collapsible cutting portion, in an expanded state, is curved. In some embodiments, the curved collapsible cutting portion is used to form a curved cut, for example when penetrating through a tissue wall that has better self-sealing properties, for example the stomach wall or any anatomical septum tissue, membrane, or tissue layer at least partly surrounding a tissue or an organ, for example epithelium layer, the skin, the diaphragm, a pleura, heart wall, and/or heart septum.

According to some exemplary embodiments, a thickness of the cut formed by the collapsible cutting portion is smaller than a maximal width, or an outer diameter of the biopsy tube.

According to some exemplary embodiments, the collapsible cutting portion of the tissue penetrator is reshaped, for example collapsed at block 106. In some embodiments, at least part of the collapsible cutting portion is collapsed at block 106. In some embodiments, the collapsible cutting portion is collapsed to acquire a width which is smaller than a minimal width of an inner lumen of the biopsy tube. In some embodiments, the cutting portion is collapsed when reaching a target tissue that needs to be sampled. Alternatively, the cutting portion is collapsed when reaching a distance smaller than 5 mm, for example smaller than 3 mm, smaller than 1 mm, smaller than 0.5 mm or any intermediate, smaller or larger distance from a target tissue that needs to be sampled.

According to some exemplary embodiments, the collapsible cutting portion is collapsed by application of force on the tissue penetrator, for example by rotating and/or retracting the tissue penetrator relative to the sampling tube. Alternatively, the collapsible cutting portion is collapsed by application of force on the sampling tube, for example by pushing, retracting and/or rotating the sampling tube relative to the tissue penetrator.

According to some exemplary embodiments, the tissue penetrator is removed from the tissue at block 108. In some embodiments, once the collapsing portion is collapsed at block 106, the tissue penetrator is removed from the tissue through the inner lumen of the sampling tube, for example by retraction of the tissue penetrator. In some embodiments, the tissue penetrator is removed from the body at block 108. In some embodiments, the tissue penetrator is removed from the inner lumen of the sampling tube, for example to allow insertion of a tissue sample into the inner lumen of the biopsy tube. In some embodiments, the tissue penetrator is retracted within the biopsy tube lumen to a distance that clears a desired volume of the biopsy tube lumen, for example a volume of at least 1 mm³, for example at least 3 mm³, at least 5 mm³, at least 1 cm³ or any intermediate, smaller or larger volume in the biopsy tube lumen, for example to allow entry of a tissue sample into the cleared volume.

### Exemplary cutting portion states

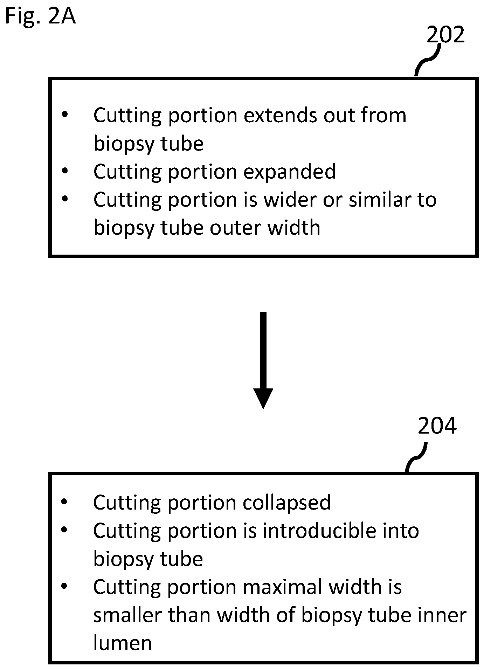

According to some exemplary embodiments, a cutting portion of a tissue penetrator moves between an expanded state and a collapsed state. In some embodiments, the collapsed state is irreversible, and for example prevents re-use of the cutting portion. Alternatively, the collapsed state is reversible, and allows return to an expanded state. In some embodiments, in a collapsed state a maximal width of the cutting portion is smaller than a minimal width of an inner lumen of a biopsy tube, for example to allow removal of the tissue penetrator from a tissue through the inner lumen of the biopsy tube. Reference is now made to fig. 2A, depicting transition between an expanded state and a collapsed state of the tissue penetrator cutting portion, according to some exemplary embodiments of the invention.

According to some exemplary embodiments, in an expanded state 202, at least part of a tissue penetrator cutting portion is expanded. In some embodiments, the cutting portion extends out from an opening of a biopsy tube inner lumen. In some embodiments, the cutting portion extends out and faces tissue located at an advancement path of the tissue penetrator. In some embodiments, in an expanded state a maximal width of the cutting portion is larger or similar to the maximal external width, for example maximal outer diameter, of the biopsy tube.

According to some exemplary embodiments, in a reshaped state, for example in a collapsed state 204, at least a portion of the tissue penetrator, for example at least part of the cutting portion is collapsed. In some embodiments, at least part of the cutting portion is collapsed to acquire a maximal width of the cutting portion that is smaller than a minimal width of the biopsy tube inner lumen. In some embodiments, in a collapsed state, the tissue penetrator is shaped and sized to be moved into the biopsy tube inner lumen.

According to some exemplary embodiments, the cutting portion of the tissue penetrator reversibly moves between an expanded state 202 to a collapsed state. Alternatively, the cutting portion irreversibly moves to a collapsed state. In some embodiments, the cutting portion of the tissue penetrator moves between an expanded state 202 and a collapsed state 204 by axially moving one or both of the biopsy tube and the tissue penetrator or a portions thereof, relative to each other. Alternatively or additionally, the cutting portion of the tissue penetrator moves between an expanded state 202 and a collapsed state 204 by rotating one or both of the biopsy tube and the tissue penetrator or portions thereof, relative to each other.

### Exemplary cut

According to some exemplary embodiments, the tissue penetrator, for example a collapsible cutting portion of the tissue penetrator is configured to perform a cut through tissue. In some embodiments, a cutting edge of the collapsible cutting portion is shaped and sized to perform a cut through a tissue wall, for example through a tissue layer, surrounding a region of tissue. In some embodiments, the tissue layer is an elastic tissue layer. For example, a tissue layer positioned between a tube, for example a sampling tube, and a target tissue that needs to be sampled. In some embodiments, the tissue wall comprises an abdomen wall, wall of the stomach, the wall of the duodenum, the wall of the intestines, the wall of the bronchial airways, blood vessel walls, GI tract walls, esophagus wall or any wall of anatomical tubes.

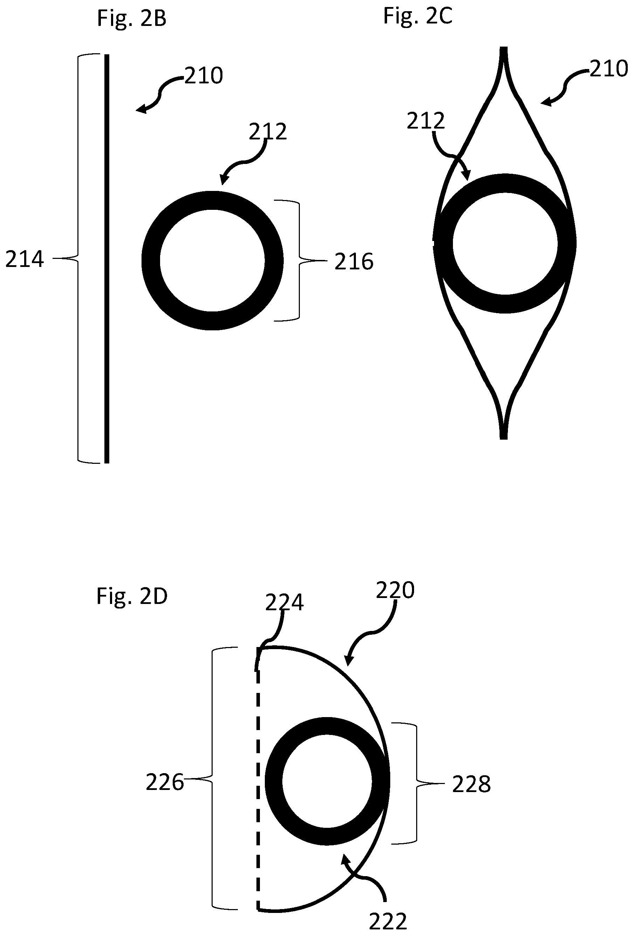

Reference is now made to figs. 2B and 2C depicting a formation of a straight cut through tissue, for example a tissue wall, according to some exemplary embodiments of the invention.

According to some exemplary embodiments, a sharp cutting edge of a tissue penetrator collapsible cutting portion is configured to form a thin straight cut through tissue, for example straight cut 210. In some embodiments, a length 214 of the straight cut 210 is in a range of 2-30 mm, for example 2-8 mm, 5-10 mm, 8-15 mm, 13-20 mm or any intermediate, shorter or longer cut. In some embodiments, a length 214 of the straight cut is equal or larger from a diameter 216 of a tube 212, for example a biopsy tube, that needs to penetrate through the straight cut. In some embodiments, a length of the cut 214 is selected according to tissue type and/or a diameter of the tube, for example on one side to be long enough to allow penetration of the tube without application of high level of forces on the tube to allow ease of use and without high level of stress on the tissue that may cause tissue ruptures, and/or in the other side not too long, for example to prevent leakage through the cut once the tube is removed from the tissue.

In some embodiments, a thickness of the straight cut 210, for example a distance between two opposite edges of the straight cut, is less than 0.5mm, for example less than 0.4 mm, less than 0.2 mm, less than 0.1 mm or any intermediate, smaller or larger value. In some embodiments, a ratio between a maximal width 214 of the straight cut 210, for example a maximal distance between two points on the cut, and a thickness of the straight cut is larger than 5:1, for example larger than 10:1, larger than 15:1, larger than 20:1, larger than 100:1 or any intermediate smaller or higher ratio. In some embodiments, the cutting edge of a tissue penetrator collapsible cutting portion forms a straight cut in a tissue wall that is less elastic and/or has low self-sealing properties, for example the intestine tissue wall or a duodenum tissue wall.

Reference is now made to figs. 2D depicting a formation of a curved cut through tissue, for example a tissue wall, according to some exemplary embodiments of the invention.

According to some exemplary embodiments, a sharp cutting edge of a tissue penetrator collapsible cutting portion is configured to form a thin curved cut, for example an arc-shaped cut, through tissue, for example curved cut 220. In some embodiments, a length of the curved cut 220 is in a range of 0.5-30 mm, for example 1-8 mm, 5-10 mm, 8-15 mm, 13-20 mm or any intermediate, shorter or longer cut. In some embodiments, a maximal width of the curved cut, for example a maximal distance between two locations on the curved cut, is larger from a diameter 228 of a tube 222, for example a biopsy tube, that needs to penetrate through the curved cut, for example as shown in fig. 2D.

According to some exemplary embodiments, a radius of curvature of said curved cut is in a range of 0.2-20 mm, for example, 0.2-8mm, 5-10mm, 7-15mm, 10-20mm or any intermediate, smaller or larger range of values. In some embodiments, the diameter of curvature of the curved cut 220 is at least an outer diameter 228 of a tube, for example tube 222 that needs to penetrate through the cut 220. In some embodiments, a length of the cut 220, and/or the radius of curvature is selected according to tissue type and/or tissue elasticity and/or a diameter of the tube, for example to allow penetration of the tube without application of high level of forces on the tissue and/or tube that may cause tissue ruptures, and/or to prevent leakage through the cut once the tube is removed from the tissue.

In some embodiments, a thickness, for example a distance between two opposite edges of the curved cut 220 is less than 0.5mm, for example less than 0.4 mm, less than 0.2 mm, less than 0.1 mm, less than 0.01 mm or any intermediate, smaller or larger value. In some embodiments, a ratio between a length or a maximal width of the curved cut 220 and a thickness of the curved cut is larger than 5:1, for example larger than 10:1, 15:1, larger than 20:1, larger than 100:1 or any intermediate smaller or higher ratio. In some embodiments, the cutting edge of a tissue penetrator collapsible cutting portion forms a curved cut in a tissue wall that is elastic and/or has high self-sealing properties, for example the stomach tissue wall.

### Exemplary tissue sampling process

According to some exemplary embodiments, a biopsy tube, for example a biopsy needle is advanced through body tissue to reach a desired target tissue that needs to be sampled directly or through a tissue wall. In some embodiments, a tissue penetrator is coupled to the biopsy tube, for example reversibly coupled, to allow easy penetration through tissue during the advancement of the biopsy tube. In some embodiments, at least part of the tissue penetrator, for example a cutting portion of the tissue penetrator is positioned in front the biopsy tube and facing the tissue.

According to some exemplary embodiments, the cutting portion of the tissue penetrator is shaped and sized to penetrate through tissue while forming a cut which is wider than a maximal width of the biopsy tube that follows the tissue penetrator. Additionally, the cut formed by the cutting has a thickness of less than 2 mm, for example less than 1mm, less than 0.1 mm or any intermediate, smaller or larger value.

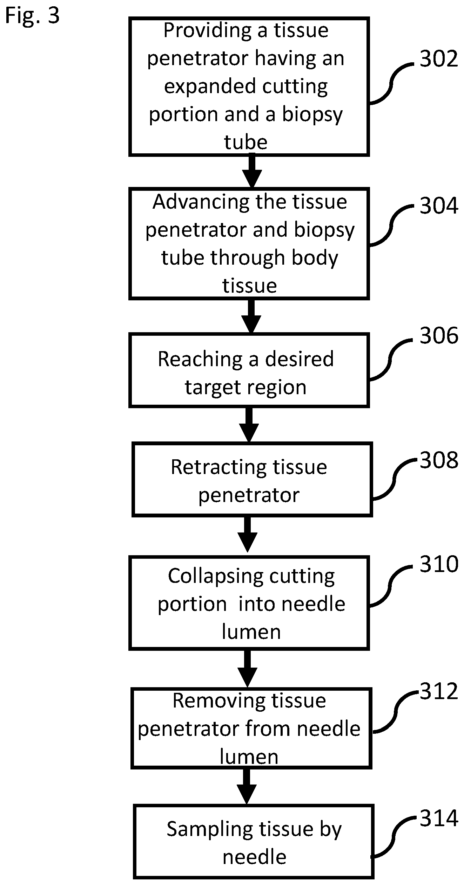

According to some exemplary embodiments, once a cut is formed, the tissue penetrator is removed from the tissue, for example through the sampling tube lumen. Reference is now made to fig. 3, depicting a tissue sampling process using a tissue penetrator, according to some exemplary embodiments of the invention.

According to some exemplary embodiments, a tissue penetrator coupled to a biopsy tube, for example a biopsy needle, is provided at block 302. In some embodiments, an assembly, for example a biopsy assembly, or a kit comprising a tissue penetrator and a biopsy tube is provided at block 302. In some embodiments, the tissue penetrator is disposed within an inner lumen of the biopsy tube. Additionally, at least part of the tissue penetrator, for example a cutting portion of the tissue penetrator extends out from the biopsy tube and positioned distally to the biopsy tube. In some embodiments, the cutting portion extends through a distal opening of an inner lumen of the biopsy tube, facing tissue.

According to some exemplary embodiments, the biopsy assembly is advanced through body tissue towards a target tissue, at block 304. In some embodiments, the biopsy assembly is advanced by applying axial force on one or both of the tissue penetrator and the biopsy tube. In some embodiments, the biopsy assembly is advanced under visualization from outside the body, for example using an imaging system, for example ultrasound, x-ray or any other imaging system.

According to some exemplary embodiments, the biopsy assembly reaches a desired target region at block 306. In some embodiments, the biopsy device reaches a desired distance from a target region, for example at a distance of up to 10 cm, up to 5 cm, up to 2 cm or any intermediate, smaller or larger distance from the target region, at block 306.

According to some exemplary embodiments, the tissue penetrator is retracted, at block 308. In some embodiments, retraction of the tissue penetrator decouples the tissue penetrator from the biopsy tube, while optionally keeping the biopsy tube at the same position. **In** some embodiments, the tissue penetrator is axially retracted relative to the biopsy tube.

According to some exemplary embodiments, at least part of the cutting portion is reshaped, for example collapsed, furled and/or rolled at block 310. In some embodiments, the reshaped cutting portion is inserted into the biopsy tube inner lumen, at block 310. In some embodiments, retraction and/or rotation of the tissue penetrator induce the reshaping, for example collapsing of the cutting edge of the tissue penetrator. In some embodiments, during reshaping the cutting edge acquires a shape and/or size that allows, for example, insertion of the reshaped cutting edge into the biopsy tube inner lumen. In some embodiments, in a reshaped state, a maximal width or diameter of the cutting portion is smaller than a minimal width or minimal diameter of an inner lumen of the biopsy tube. In some embodiments, the reshaping of the cutting portion and insertion of the cutting portion into the biopsy tube lumen are performed simultaneously. Alternatively, the reshaping of the cutting portion and insertion of the cutting portion into the biopsy tube lumen, are performed sequentially, for example by applying forces in different directions and/or in two separate time points.

According to some exemplary embodiments, the tissue penetrator is removed from the inner lumen of the biopsy tube, for example biopsy needle, at block 312. In some embodiments, the tissue penetrator is removed from the inner lumen, for example to allow entry of tissue sample into the inner lumen. In some embodiments, the tissue penetrator is removed by retraction of the tissue penetrator within the inner lumen.

According to some exemplary embodiments, the tissue penetrator is removed completely from the inner lumen of the sampling tube, and optionally outside the body. Alternatively, the tissue penetrator is partly removed, for example to evacuate a predetermined volume of the biopsy tube lumen. In some embodiments, evacuating the predetermined volume allows, for example, to sample a predetermined tissue sample volume.

According to some exemplary embodiments, body tissue is sampled at block 314. In some embodiments, during body tissue sampling the biopsy tube, for example biopsy needle is advanced into the body tissue while a sample of the body tissue is pushed into the inner lumen of the biopsy tube. In some embodiments, during the advancement of the biopsy tube, for example by rotation of the biopsy tube, a tissue sample enters into a volume of the biopsy tube inner lumen, for example a volume evacuated at block 312 by removal of the tissue penetrator.

### Exemplary tissue penetrator and biopsy assembly

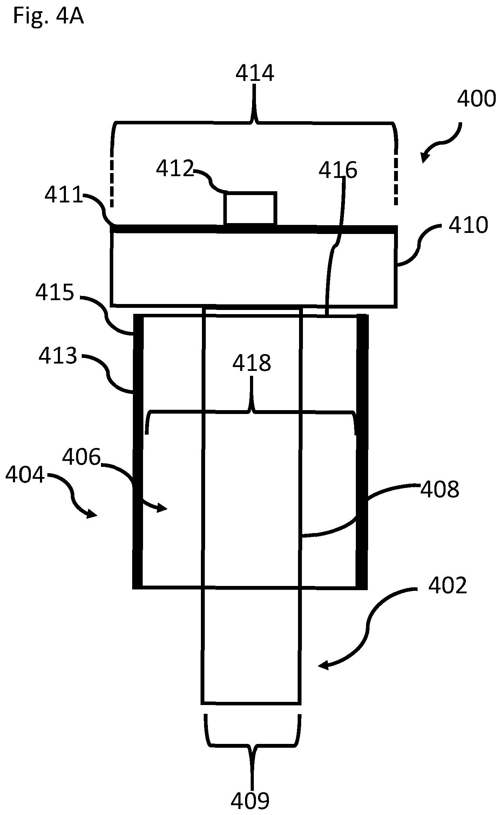

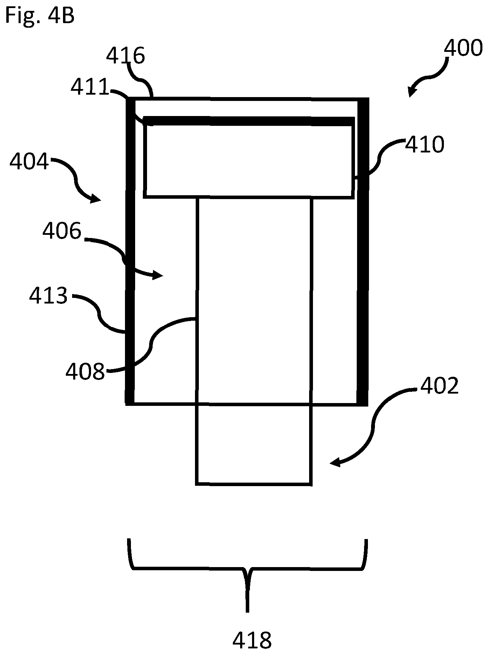

Reference is now made to figs. 4A and 4B depicting a biopsy assembly comprising a biopsy tube and a tissue penetrator, according to some exemplary embodiments of the invention.

According to some exemplary embodiments, a biopsy assembly, for example assembly 400, comprises a tissue penetrator 402 and a biopsy tube 404. In some embodiments, the tissue penetrator 402 is reversibly coupled to the biopsy tube 404. In some embodiments, for example as shown in figs. 4A and 4B, at least part of the tissue penetrator is disposed within an inner lumen 406 of the biopsy tube 404.

According to some exemplary embodiments, a tissue penetrator, for example tissue penetrator 402 comprises an elongated body 408 and a cutting portion 410 located at a distal section of the elongated body, for example a section that is closer to body tissue. Optionally, the cutting portion is located at a distal end of the elongated body, for example an end of the elongated body closer to body tissue. Optionally, the tissue penetrator comprises a tip 412, for example a conical tip. In some embodiments, the tip 412 is at least partly beveled. In some embodiments, the tip 412 is shaped and sized to allow easy penetration through body tissue, for example as the tissue penetrator advances through body tissue. In some embodiments, the tip 412 is part of the cutting portion 410. Alternatively, the tip 412 is located distally to the cutting portion 410.

According to some exemplary embodiments, the cutting portion 410, for example a collapsible cutting portion, comprises a cutting edge 411. In some embodiments, the cutting edge 411 is located on a distal surface of the cutting portion 410 facing a tissue. Optionally, the cutting edge 411 is a leading edge of the cutting portion 410.

According to some exemplary embodiments, the cutting edge 411 is sharp. Additionally or alternatively, in some embodiments, the cutting portion is thin, for example has a thickness of less than 0.5 mm, for example less than 0.3 mm, less than 0.1 mm or any intermediate, smaller or larger value. In some embodiments, a length of the cutting edge 411 is in a range of 2-50mm, for example 5-10mm, 8-15mm, 12-20mm, 15-30mm or any intermediate, smaller or larger range of values. In some embodiments, a ratio between a length or maximal width, for example a maximal distance between two points on the cutting edge, and a thickness of the cutting edge is at least 10:1, for example at least 20:1, at least 30:1 or any intermediate, smaller or larger ratio value. In some embodiments, a ratio between a length of the cutting edge 411 and the maximal width/ diameter of the cutting edge is at least 2:1, for example at least 5:1, at least 20:1 or any intermediate, smaller or larger ratio.

According to some exemplary embodiments, the cutting edge 411 is planar, for example straight. In some embodiments, the planar cutting edge is used to perform a straight cut, for example as described in figs. 2B and 2C.

According to some exemplary embodiments, the cutting edge 411 is curved. In some embodiments, the curved cutting edge has a radius of curvature in a range of 0.2-20mm, for example 0.2-5mm, 5-10mm, 7-15mm, 10-20mm or any intermediate, smaller or larger range of values.

According to some exemplary embodiments, a specific tissue penetrator with a specific type of cutting edge is selected according to the tissue type that needs to be cut, for example a tissue penetrator with a planar cutting edge is selected, when a cut needs to be performed in a tissue wall with low elasticity, for example a tissue wall of the intestine or the duodenum. Alternatively, a tissue penetrator with a curved cutting edge is selected, when a cut needs to be performed in an elastic tissue, for example a wall of the stomach. Alternatively or additionally, a specific type of penetrator with a specific cutting edge is selected according to the external diameter of the tube that needs to penetrate through the formed cut.

According to some exemplary embodiments, the elongated body 408 comprises a shaft. In some embodiments, the elongated body 408 is flexible and bendable, for example to allow bending while at least one of, the assembly 400, the biopsy tube 404 and/or the tissue penetrator 402 is steered towards a desired target region in the body. In some embodiments, the elongated body is bendable in at least 40° degrees, for example at least 45°degrees, at least 60°degrees, at least 70° degrees or any intermediate, smaller or larger angle. In some embodiments, the elongated body is formed from stainless steel, steel, steel alloy, e.g. cobalt chromium, a super elastic alloy, e.g. NiTi, or a shape memory alloy.

According to some exemplary embodiments, a width 409 of the elongated body 408, is up to 95%, for example up to 90%, up to 80% or any intermediate, smaller or larger percentage value, of the width 418 of the inner lumen 406, for example to allow the tissue penetrator 402 to support the biopsy tube 404 during navigation to a desired target region. In some embodiments, a thickness of a wall 413 surrounding said inner lumen 406 is at least 5%, for example at least 10%, at least 20%, at least 30% or any intermediate, smaller or larger percentage value of the inner width or the inner diameter of the inner lumen 406 of the biopsy tube.

According to some exemplary embodiments, a maximal width or a maximal diameter of the tissue penetrator body 408 is in a range of 0.30-7mm, for example 0.3-1mm, 0.8-2mm, 1.5-3mm, 2-4mm, 3.5-5mm, 4.5-7mm or any intermediate, smaller or larger range of values. In some embodiments, a maximal width or a maximal diameter of the inner lumen 406 is larger in at least 0.02 mm, for example at least 0.5mm, at least 1 mm, at least 3 mm or any intermediate, smaller or larger value, from the maximal diameter of the tissue penetrator body.

According to some exemplary embodiments, the cutting portion 410 is a re-shapeable cutting portion and is configured to move from an expanded state to a collapsed state, for example as described in fig. 2A. In some embodiments, in an expanded state, for example as shown in fig. 4A, the cutting portion is located outside and distal to the biopsy tube 404. In some embodiments, the cutting portion 410 is mechanically connected to a portion of the body 408 extending out from the biopsy tube lumen 406, for example through a distal opening 416. Alternatively, the cutting portion 410 is an integral part of the body 408.

According to some exemplary embodiments, in an expanded state, for example as shown in fig. 4A, a maximal width 414 of the cutting portion 410 is larger than a maximal width 418 or a maximal diameter of the inner lumen 406. In some embodiments, in the expanded state, a maximal width 414 of the cutting portion 410 is larger than a maximal width or a maximal diameter of the inner lumen distal opening 416. In some embodiments, the maximal width 414 of the cutting portion 410 is in a range of 0.40-8mm.

According to some exemplary embodiments, in an expanded state the cutting portion comprises a single continuous distal cutting edge, facing tissue. Alternatively, the cutting portion comprises two or more distally extending sections, each having a distal cutting edge facing tissue. Alternatively, the cutting portion comprises two or more distally extending sections of a single continuous cutting edge. In some embodiments, the cutting portion distally extends to form the tip 412.

According to some exemplary embodiments, the cutting portion is reshaped to enter the inner lumen 406 of the biopsy tube, for example as shown in fig. 4B. In some embodiments, reshaping of the cutting portion is induced by retracting and/or rotating the tissue penetrator 402, for example the tissue penetrator body 408. In some embodiments, the tissue penetrator body is retracted and/or rotated from outside the body. In some embodiments, in a reshaped, for example a collapsed state, a maximal width or diameter of the cutting portion is smaller than an internal width or diameter of the inner lumen 406, for example to allow removal of the tissue penetrator 402 from the body through the biopsy tube inner lumen, for example as described at block 312 of fig. 3.

According to some exemplary embodiments, retraction and/or rotation of the tissue penetrator body 408 induce furling or rolling of at least part of the cutting portion 410, for example to acquire a desired shape which has smaller dimensions, for example width or diameter, relative to the dimensions of the inner lumen 406. Alternatively or additionally, retraction and/or rotation of the tissue penetrator body 408 induce at least part of the cutting portion to collapse, for example to acquire the desired shape. In some embodiments, retraction and/or rotation of the tissue penetrator body 408 induce two or more ends of the cutting portion to bend until an overlap is formed.

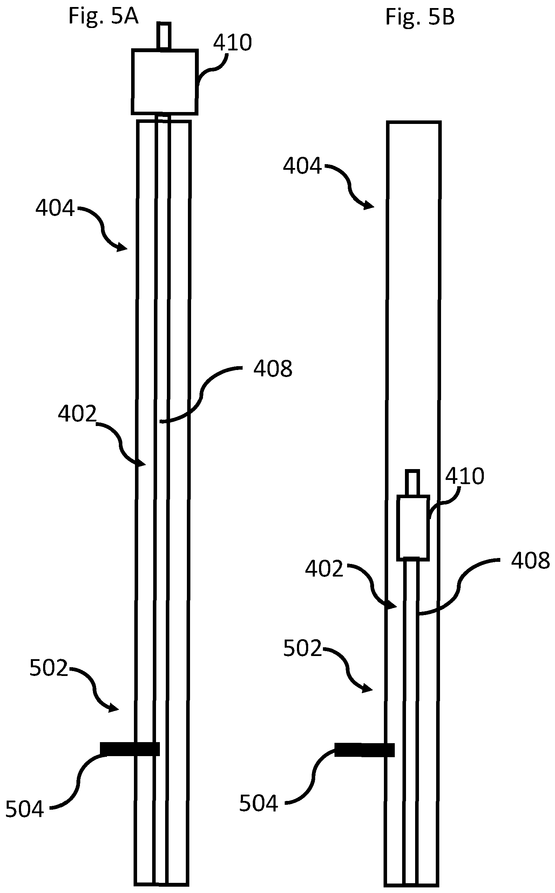

Reference is now made to figs. 5A and 5B depicting coupling between a tissue penetrator and a biopsy tube, according to some exemplary embodiments of the invention.

According to some exemplary embodiments, the tissue penetrator is coupled to the biopsy tube, for example during the navigation of the biopsy assembly to a target region in the body. In some embodiments, the tissue penetrator is reversibly coupled to the biopsy tube, for example to allow removal of the tissue penetrator prior to or during tissue sampling.

According to some exemplary embodiments, coupling of the tissue penetrator to the biopsy tube allows the biopsy tube to support the distally extending cutting against external pressure applied on the cutting portion, for example during the navigation. In some embodiments, for example as shown in fig. 4A, during navigation, the cutting portion 410 is pressed against a distal end of the biopsy tube body 415 that surrounds, for example, an edge of the opening 416 by forces applied by the tissue. In some embodiments, the cutting portion is shaped and configured to be pressed against the body 415 without collapsing.

According to some exemplary embodiments, a minimal thickness of a wall of said biopsy tube body is at least 5%, for example at least 10%, at least 15%, at least 20% or any intermediate, smaller or larger percentage value, of the inner diameter of the biopsy tube. In some embodiments, a minimal thickness of a wall of said biopsy tube body is at least 0.03 mm, for example at least 0.05 mm, at least 0.07 mm, at least 0.1 mm or any intermediate, smaller or larger value.

According to some exemplary embodiments, for example as shown in fig. 5A, the coupling between the tissue penetrator 402 and the biopsy tube 404 is generated by a coupler 504 located at a proximal section 502 of the biopsy tube 404. In some embodiments, the coupler 504 mechanically couples the biopsy tube 404 and the tissue penetrator 402 during navigation towards a target region. In some embodiments, the coupler fixes a position of the tissue penetrator 402 relative to the biopsy tube 404 in an axial direction, while optionally allowing rotation of the biopsy tube and/or the tissue penetrator relative to each other. In some embodiments, the coupler 504 comprises a reversible lock, for example a reversible interlocking mechanism. In some embodiments, forces applied on the cutting portion press the tissue penetrator against the interlocking mechanism. In some embodiments, the interlocking mechanism transfers at least part of the applied force to the biopsy tube.

According to some exemplary embodiments, for example as shown in fig. 5B, release of the interlocking mechanism allows, for example reshaping of the cutting portion and/or retraction of the tissue penetrator 402 through the lumen of the biopsy tube 502. In some embodiments, decoupling of the tissue penetrator 402 from the biopsy tube 404 is irreversible. Optionally, the coupler 504 is part of a mechanism for rotation and/or retraction of the tissue penetrator to allow, for example reshaping of the cutting portion.

### Exemplary tissue penetration

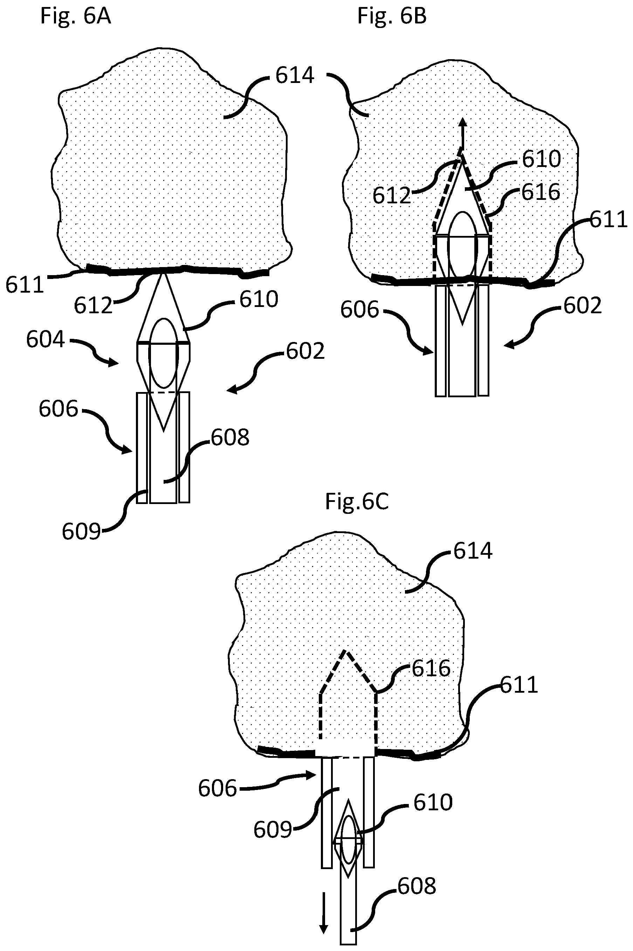

According to some exemplary embodiments, a biopsy assembly, comprising a biopsy tube and a tissue penetrator is advanced through body tissue towards a desired target region. In some embodiments, at least part of the tissue penetrator, for example a cutting portion, is located distally to the biopsy tube, and is shaped and sized to penetrate and perform a cut, as the assembly advances towards the target region. In some embodiments, the cut formed by the cutting portion allows, for example easy penetration of the biopsy tube located proximally to the cutting portion, through the tissue. Reference is now made to figs. 6A-6E, describing penetration through tissue, according to some exemplary embodiments of the invention.

According to some exemplary embodiments, a biopsy assembly 602 comprises a tissue penetrator 604 and a biopsy tube 606. In some embodiments, prior to penetration through tissue, the tissue penetrator body 608 is located within an inner lumen 609 of the biopsy, and a cutting portion 610 of the tissue penetrator 604 connected to the body 608, distally extends out from the biopsy tube 606. In some embodiments, when the cutting portion extends out from the biopsy tube, the cutting portion is in an expanded state, where a maximal width of the cutting portion is larger or has a width similar to the biopsy tube outer width, for example outer diameter.

According to some exemplary embodiments, the tissue penetrator comprises a distal tip, for example a sharp distal tip 612, located distally to the cutting portion. In some embodiments, the distal tip is an integral part of the cutting portion 610. Alternatively, the distal tip is an integral part of the body 608, for example in embodiments in which the body extends out from the biopsy tube and is connected to the cutting portion.

According to some exemplary embodiments, for example as shown in fig. 6B, as the assembly 602 advances into and through tissue 614, the tip 612 penetrates through the tissue 614, while the expanded cutting portion 610 forms a thin cut 616 which is wider or has a width similar to the width of the proximally positioned biopsy tube 606. In some embodiments, the tissue applies force on the cutting portion, as the tissue penetrator advances through the tissue. In some embodiments, the force applied on the cutting portion pushes the cutting portion against the biopsy tube, which structurally supports the cutting portion without inducing reshaping, for example collapse of the cutting portion. Alternatively, a coupler, for example coupler 504 shown in figs. 5A and 5B, structurally supports the tissue penetrator body against the forces applied on the cutting portion 610.

According to some exemplary embodiments, for example as shown in fig. 6B, once the assembly 602 has reached a desired target region in the tissue, the tissue penetrator 604 is removed from the tissue 614. In some embodiments, when the tissue penetrator 604 is removed, the biopsy tube 606 remains at the same position. In some embodiments, the cutting portion 610 is reshaped, for example collapsed, for example to acquire a shape and/or size that can fit into the inner lumen 609 of the biopsy tube 606.

According to some exemplary embodiments, for example as shown in fig. 6C, the cutting portion 610 is reshaped, for example collapsed, by retraction and/or rotation of the body 608. In some embodiments, retraction of the tissue penetrator body 608 causes the cutting portion to collapse, for example during and/or prior to entry into the biopsy tube lumen 609.

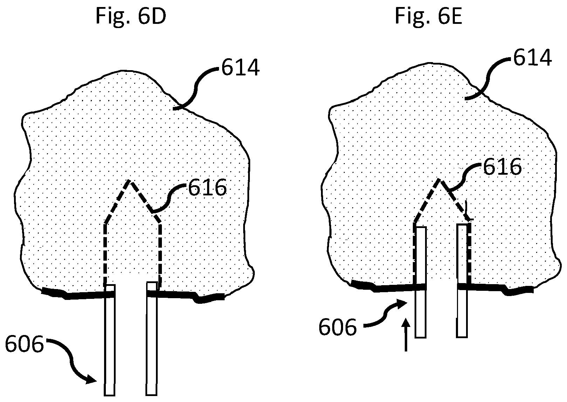

According to some exemplary embodiments, the tissue penetrator is removed from the body, for example as shown in fig. 6D. In some embodiments, the biopsy tube is advanced into the tissue, and at least partly through the cut 616, for example as shown in fig. 6E. In some embodiments, the cut 616 allows, for example, easy entry of the biopsy tube through the tissue wall 611. Alternatively or additionally, the cut shape and/or size allows self-sealing by the tissue, for example to prevent leakage through the cut in the tissue wall when the biopsy tube is retracted.

### Exemplary cutting portion reshaping

According to some exemplary embodiments, a tissue penetrator comprises longitudinal axis, a distal section shaped and sized to contact a tissue, and a proximal section. In some embodiments, a collapsible cutting portion, configured to reshape and acquire a narrow shape, for example a narrow shape that fits into an internal lumen of a biopsy tube, is located at the distal section. In some embodiments, the cutting portion comprises a cutting edge on an external surface of the cutting portion facing a tissue. In some embodiments, an elongated body connected to the cutting portion is located at the proximal section. Optionally, the cutting portion is integrated with the elongated body.

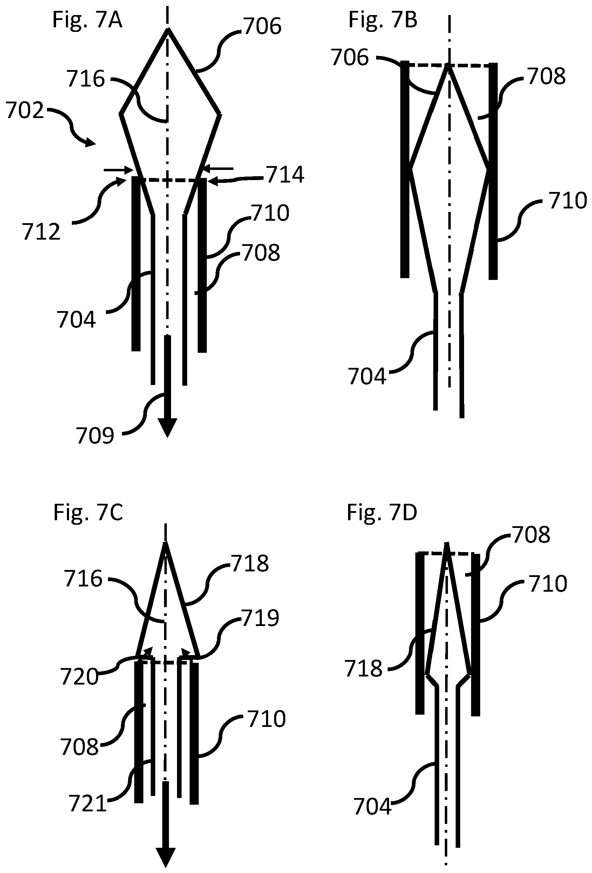

According to some exemplary embodiments, the cutting portion is reshaped by forces applied by a biopsy tube, in which the tissue penetrator body is disposed, on the cutting portion. In some embodiments, the cutting portion, is bendable, for example to allow collapse of the cutting portion. Optionally, the cutting portion is elastic, for example to allow collapse of the cutting portion and return to a previous expanded shape. Reference is now made to figs. 7A-7F, depicting reshaping of a cutting portion, according to some exemplary embodiments of the invention.

According to some exemplary embodiments, a tissue penetrator, for example tissue penetrator 702 comprises a proximal elongated body 704, a distal cutting portion 706 connected to the elongated body, and a longitudinal axis 716. In some embodiments, the tissue penetrator 702 is disposed at least partly within an inner lumen 708 of a biopsy tube 710. In some embodiments, for example, as shown in fig. 7A, the cutting portion distally extends at least partly from the inner lumen 708.

According to some exemplary embodiments, a cross section or a projection of the cutting portion, for example cutting portion 706 is a rhombus, a deltoid, a deltoid with curved or angled corners or a diamond shape. In some embodiments, the cutting portion is a collapsible cutting portion, and configured to inwardly collapse towards the longitudinal axis 716 of the tissue penetrator. In some embodiments, the cutting portion is configured to inwardly collapse, in response to an inward force applied on opposite sides of the cutting portion 706, for example by the biopsy tube 710, at one or more contact points, for example contact points 712 and 714 between the cutting portion 706 and the biopsy tube 710.

According to some exemplary embodiments, for example as shown in fig. 7B, the inward collapse reshapes the cutting portion 706 to have a maximal width smaller than a minimal width of the biopsy tube inner lumen 708.

According to some exemplary embodiments, during the advancement of the tissue penetrator 702 and the biopsy tube 710 through tissue, the biopsy tube 710 supports the cutting portion 706, at one or more contact points, against force applied by the tissue on the cutting portion 706. In some embodiments, the force applied by the tissue is lower than a force value needed to induce reshaping. In some embodiments, the cutting portion shape is configured to resist a force value of up to 0.5-5 Newton (N), for example 0.5-2N, 1-3N 2-5N or any intermediate, smaller or larger range of values, without reshaping, for example by leaning against the biopsy tube 710. In some embodiments, retraction of the tissue penetrator exceeds this force value, leading to reshaping of the cutting portion.

According to some exemplary embodiments, for example as shown in fig. 7C, a cutting portion 718 has a cross-section shaped as a triangle, in which a base of the triangle 719 leans against the biopsy tube body 710, for example during advancement through body tissue.

According to some exemplary embodiments, a wall 720 of the tissue penetrator is positioned at angle of less than 20 degrees, for example less than 15 degrees, less than 10 degrees or any intermediate, smaller or larger degree between the tissue penetrator wall and the tissue penetrator body 721. In some embodiments, the wall 720 leans against the biopsy tube 710, for example to support the cutting portion 718 against forces applied by tissue on the cutting portion as the cutting portion advances through the tissue, without causing reshaping of the cutting portion.

According to some exemplary embodiments, retraction of the body 721, applies force by the biopsy tube 710 on the cutting portion 718, leading to an inward collapse of the cutting portion 718, for example towards the longitudinal axis 716. In some embodiments, for example as shown in fig.7D, in a collapsed state, a maximal width of the cutting portion is smaller than a minimal width of the biopsy tube inner lumen 708.

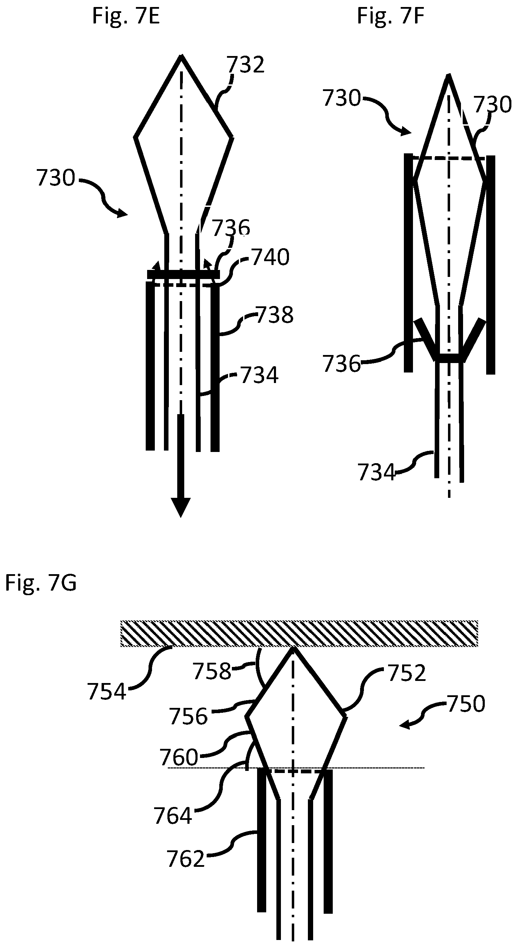

According to some exemplary embodiments, for example as shown in fig. 7E, a tissue penetrator comprises a distal cutting portion 732 connected to a proximal body 734, or is integrated with the body 734. In some embodiments, the tissue penetrator 730 comprises a stopper 736, located proximal to the cutting portion 732, for example between the cutting portion 732 and a biopsy tube body 738, when the cutting portion is in an expanded state. In some embodiments, during the advancement of the tissue penetrator 730 through tissue, forces applied by the tissue on the cutting portion 732 push the stopper 736 against the body 738.

According to some exemplary embodiments, the stopper 736 is configured to resist pressure applied by the biopsy tube body 738 on the tissue penetrator during advancement through tissue, without causing the cutting portion 732 to reshape, for example collapse. In some embodiments, the stopper is configured to prevent direct contact between the biopsy tube body 738 and the cutting portion 732. In some embodiments, for example during the advancement through tissue, the stopper is reversibly mechanically coupled to the biopsy tube body 738. In some embodiments, the stopper is coupled to the tissue penetrator body 734.

According to some exemplary embodiments, retraction of the tissue penetrator 730, for example as shown in fig. 7F applies a force of the stopper 736 which is larger than a force the stopper 736 can resist. In some embodiments, the force applied on the stopper 736 during retraction of the tissue penetrator 730 relative to the biopsy tube 738, deforms the stopper 736. In some embodiments, deformation of the stopper leads to direct contact between the biopsy tube 738 and the cutting portion 730, for example a collapsible cutting portion, causing the cutting portion 730 to collapse, for example as described in fig. 7B. In some embodiments, the stopper is configured to resist pressure of up to 0.5-5N, for example 0.5-2N, 1-3N. 2.5-4N, 3.5-5N or any intermediate, smaller or larger range of values.

Reference is now made to fig. 7G depicting a penetration angle between a cutting portion of a tissue penetrator and a tissue wall, and an angle between the cutting portion and the biopsy tube, according to some exemplary embodiments of the invention.

According to some exemplary embodiments, a tissue penetrator, for example a tissue penetrator 750 comprising a cutting portion 752, for example a collapsible portion. In some embodiments, at least one external surface of the cutting portion 752 is a non-planar surface, for example a curved or an angled external surface. In some embodiments, at least one external surface of the cutting portion 752 facing a tissue, for example tissue wall 754 is non-planar, for example surface 756.

According to some exemplary embodiments, an external surface of the cutting portion 752 facing a tissue, for example surface 756 is positioned at angle 758 larger than 45 degrees, for example at an angle larger than 50 degrees, larger than 60 degrees, larger than 70 degrees, larger than 87 degrees or any intermediate, smaller or larger angle between the surface 756 and tissue wall 754. In some embodiments, an angle larger than 45 degrees between an external surface of the cutting portion facing the tissue and the tissue allows, for example to penetrate into the tissue while applying low axial forces by the cutting portion 752 on the tissue 754.

According to some exemplary embodiments, an external surface of the cutting portion 752, for example surface 760 facing a biopsy tube 762 in which the tissue penetrator is disposed, is positioned at an angle relative to the biopsy tube 762. In some embodiments, an angle 764 between the biopsy tube 762 and the surface 760 is larger than 45 degrees, for example larger than 50 degrees, larger than 60 degrees, larger than 70 degrees, larger than 87 degrees or any intermediate, smaller or larger angle between the surface 760 and the biopsy tube 762. In some embodiments, an angle larger than 45 degrees between an external surface of the cutting portion facing the biopsy tube and the tissue biopsy tube allows, for example, to collapse the cutting portion by applying relatively low axial forces on the penetrator body 734 by the biopsy tube 762, for example by retracting the penetrator body into an inner lumen of the biopsy tube.

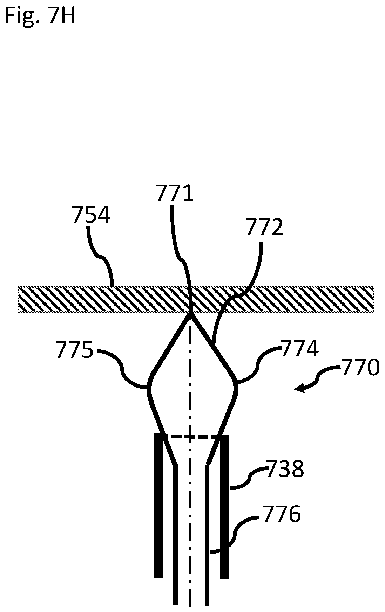

Reference is now made to fig. 7H, depicting a tissue penetrator with curved lateral walls, according to some exemplary embodiments of the invention.

According to some exemplary embodiments, a tissue penetrator, for example tissue penetrator 770 comprises a cutting portion 772, for example a collapsible cutting portion, connected to an elongated body 776. In some embodiments, the cutting portion 772 comprises a distal tip, for example distal tip 771 connected to the elongated body 776 via lateral surfaces 774 and 775, for example side surface. In some embodiments, for example as shown in fig. 7H, the lateral surfaces 774 and 775 are curved, for example non-angled. In some embodiments, the lateral surfaces are curved, when the cutting portion is in an expanded state, for example when the cutting portion extends out from the inner lumen of the biopsy tube body 738. Optionally, the distal tip of the cutting portion is curved. Optionally, an external edge of the cutting portion is entirely curved. Alternatively, at least part of the external edge is curved.

### Exemplary tissue penetrator having a curved cutting portion

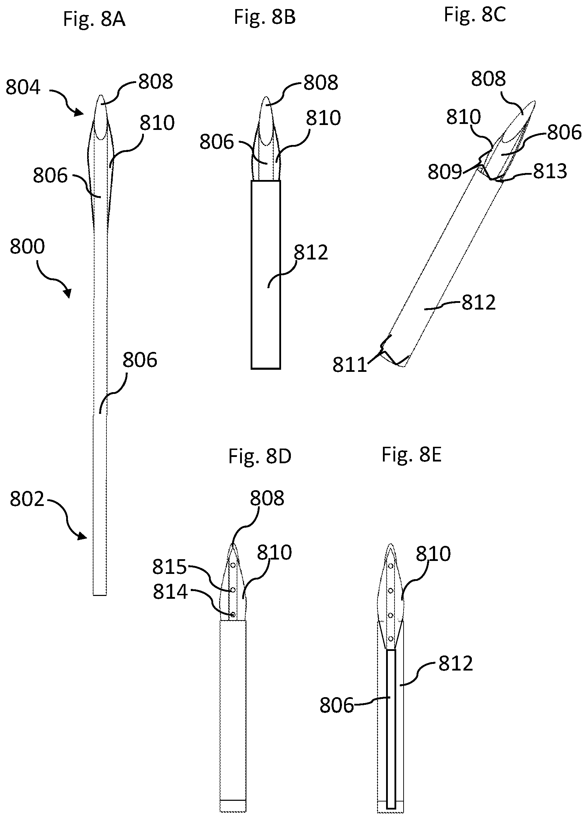

Reference is now made to figs. 8A-8E depicting a tissue penetrator with a curved cutting portion, according to some exemplary embodiments.

According to some exemplary embodiments, a tissue penetrator 800 comprises an elongated body 806 having a proximal section 802 and a distal section 804. In some embodiments, the elongated body 806 comprises an elongated shaft. In some embodiments, the shaft is flexible, for example to allow bending of the tissue penetrator while advancing through a sleeve, for example an endoscope. In some embodiments, the elongated shaft comprises a distal tip 808. In some embodiments, the distal tip 808 is closed, for example to prevent penetration of tissue into the shaft. Optionally, the distal tip 808 is beveled. Optionally or additionally, the distal tip 808 is at least partly sharpened, for example to allow easy penetration through tissue.

According to some exemplary embodiments, a cutter, for example a cutting portion 810 is mechanically connected to a distal section 804 of the elongated body 806, for example to a distal section of the shaft. In some embodiments, the distal tip 808 is positioned distally to the cutting portion 810. In some embodiments, the cutting portion 810 is at least partly curved.

According to some exemplary embodiments, the cutting portion 810 has a curved surface, which is curved around a longitudinal axis of the tissue penetrator, for example around a longitudinal axis of the elongated body 806. In some embodiments, a maximal length of the curved surface perpendicular to the elongated body is larger than a width of the elongated body. In some embodiments, an arc length of the curved surface is fixed. Alternatively, the arc length varies along a longitudinal axis of the body 806. In some embodiments, a maximal arc length of the curved surface is in a range of 0.8-20mm, for example 0.8-3mm, 1-5mm, 3-7mm, 6-10mm, 8-15mm, 13-20mm or any intermediate, smaller or larger range of values. In some embodiments, a ratio between an overall width of the curved surface 810 of the cutting portion relative to the width or diameter of elongated body 806, is in a range between a 1:1 ratio and a 15:1 ratio, respectively. In some embodiments, a maximal length of the cutting portion 810 along the longitudinal axis of the body 806 is in a range of 1-60mm, for example 1-10mm, 8-20mm, 15-30mm, 20-40mm, 30-50mm, 40-60mm or any intermediate, smaller or larger range of values.

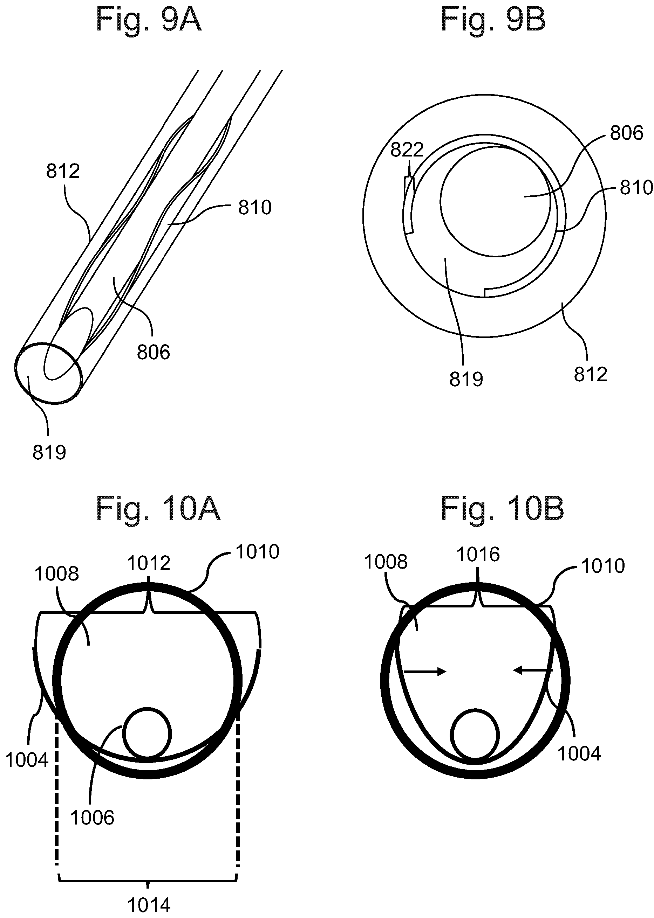

According to some exemplary embodiments, an external edge of the cutting portion 810 which extends on both sides of the elongated body 806 is at least partly sharpened, for example to allow cutting of tissue as the tissue penetrator advances axially into the tissue. In some embodiments, a maximal thickness 822 of the cutting portion 810, for example as shown in fig. 9B, is smaller than 0.1 mm, for example smaller than 0.8 mm, smaller than 0.6 mm, smaller than 0.5 mm or any intermediate, smaller or larger value. In some embodiments, the thickness 822 is calculated by reducing a width of the body 806 from the width of the internal lumen 819, and dividing the result in at least 2, for example at least 3, at least 4 or any intermediate, smaller or larger value.

According to some exemplary embodiments, for example as shown in figs. 8D and 8E, the cutting portion 810 is coupled to the elongated body 806, at one or more contact points along the body 806. In some embodiments, the cutting portion 810 is coupled to the elongated body by welding at one or more welding points, for example welding points 814 and 815. Alternatively, the cutting portion is coupled to the elongated body by soldering, gluing, crimping and any other bonding options of metal parts.

According to some exemplary embodiments, for example as shown in figs. 8B-8E, the tissue penetrator is shaped and sized to be positioned at least partly within a biopsy tube 812, which is optionally a biopsy needle. In some embodiments, the tissue penetrator, for example the cutting portion 810 and the body 806 extend distally from the tube 812, for example through a distal opening 813 of the tube 812. In some embodiments, for example as shown in fig. 8C, a maximal width of the cutting portion 810, for example when the cutting portion is expanded, is smaller or equal to the external width 811 of the biopsy tube 812. In some embodiments, when the cutting portion is expanded, a maximal width of the cutting portion is larger than the width of the biopsy tube inner lumen.

Reference is now made to figs. 9A and 9B, depicting a cutting portion in a collapsed state, for example in a folded state, according to some exemplary embodiments of the invention.

According to some exemplary embodiments, in a collapsed state, the tissue penetrator is inserted into the inner lumen 819 of the biopsy tube 812. In some embodiments, the cutting portion 810 is folded, for example at least partly around the body 806. In some embodiments, the cutting portion 810 is flexible, for example to allow folding in a collapsed state. In some embodiments, the cutting portion 810 is irreversible collapsible, for example the cutting portion 810 cannot return to a previous shape once inwardly collapsed. Optionally, the cutting portion is also elastic, for example to allow return to a previous expanded state.

According to some exemplary embodiments, in a collapsed state, for example as shown in fig. 9B, the cutting portion contacts the inner surface of the biopsy tube lumen 819. Optionally, in a collapsed state, sharpened edges of the cutting portion are positioned at a distance from the inner surface of the lumen 819, for example to prevent damage to the surface as the tissue penetrator moves within the lumen 819.

Reference is now made to figs. 10A-10D depicting collapse of a curved cutting portion, according to some exemplary embodiments of the invention.

According to some exemplary embodiments, for example as shown in fig. 10A, a curved cutting portion 1004 is coupled to a tissue penetrator body, for example shaft 1006. In some embodiments, in an expanded state shown in fig. 10A, a maximal width 1012 of the cutting portion 1004 is larger than a width 1014 of a biopsy tube 1010 inner lumen 1008, in which the tissue penetrator is at least partly located. In some embodiments, in an expanded state, the cutting portion extends at least partly out from the inner lumen 1008.

According to some exemplary embodiments, for example shown in fig. 10B, the cutting portion 1004 is configured to collapse, for example to fold, to acquire a width 1016 which is smaller than the width of the inner lumen 1008. In some embodiments, the cutting portion is elastic enough to bend inwardly, for example to acquire a width which is smaller than the inner lumen 1008 width.

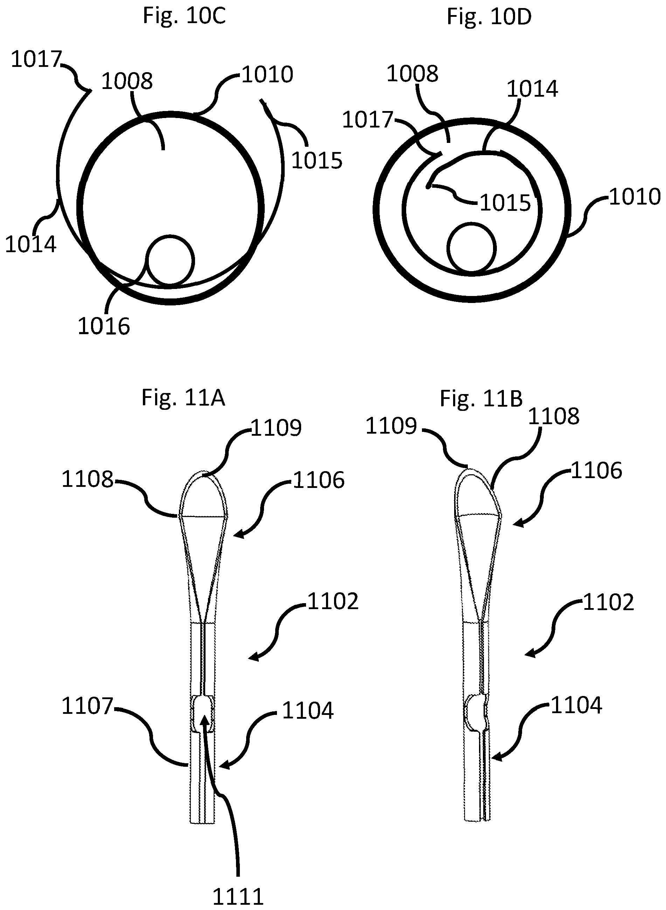

Alternatively, for example as shown in figs. 10C and 10D, a curved cutting portion 1014 coupled to tissue penetrator body 1016 is configured to furl, for example to roll, at least partly within the lumen 1008. In some embodiments, when the cutting portion 1014 is at least partly rolled, two opposite ends, for example opposite ends 1017 and 1015 overlap and optionally contact each other. Alternatively, two opposite ends of the cutting portion are at least partly rolled without overlap, for example as shown in fig. 9B.

### Exemplary tissue penetrator with an integrated cutting portion

Reference is now made to figs. 11A and 11B, depicting a tissue penetrator with an integrated cutting portion, according to some exemplary embodiments of the invention.

According to some exemplary embodiments, a tissue penetrator 1102 comprises a proximal section 1104 and a distal section 1106. In some embodiments, the tissue penetrator comprises an elongated body 1107 at the proximal section, and a cutting portion 1108 at the distal section 1106. In some embodiments, the cutting portion 1108 is integrated with the body, for example formed from the same sheet or layer of material.

According to some exemplary embodiments, the body 1107 and cutting portion 1108 are formed from a single layer of material, for example from a sheet of steel, stainless steel, steel alloy e.g. cobalt chromium, or super elastic alloy, a shape memory alloy, for example Nitinol. In some embodiments, the body 1107 is formed by bending the layer to form a tubular shape, for example an enclosed tube, which is shaped and sized to be placed inside a biopsy tube. In some embodiments, the cutting portion is formed by cutting a tube, for example using laser cutting. In some embodiments, a portion of the body that is shaped and sized to be positioned inside the biopsy tube has a fixed width or diameter, which is smaller than the width or diameter of the biopsy tube. Additionally, the external surface of the body 1107 is smooth, for example to prevent damage to the inner surface of the biopsy tube.

According to some exemplary embodiments, the cutting portion is formed by bending the layer of material to form a concave shape, with optionally a distally extending tip 1109. In some embodiments, a maximal width of the cutting portion 1108 is larger than a maximal width of the body 1107.

According to some exemplary embodiments, a body 1107 comprises a window or opening, for example opening 1111, for example to allow to lower the retraction force.

It is expected that during the life of a patent maturing from this application many relevant biopsy tubes will be developed; the scope of the term biopsy tube is intended to include all such new technologies *a priori.*

As used herein with reference to quantity or value, the term "about" means "within ± 20 % of".

The terms "comprises", "comprising", "includes", "including", "has", "having" and their conjugates mean "including but not limited to".

The term "consisting of" means "including and limited to".

The term "consisting essentially of" means that the composition, method or structure may include additional ingredients, steps and/or parts, but only if the additional ingredients, steps and/or parts do not materially alter the basic and novel characteristics of the claimed composition, method or structure.

As used herein, the singular forms "a", "an" and "the" include plural references unless the context clearly dictates otherwise. For example, the term "a compound" or "at least one compound" may include a plurality of compounds, including mixtures thereof.

Throughout this application, embodiments of this disclosure may be presented with reference to a range format. It should be understood that the description in range format is merely for convenience and brevity and should not be construed as an inflexible limitation on the scope of the invention. Accordingly, the description of a range should be considered to have specifically disclosed all the possible subranges as well as individual numerical values within that range. For example, description of a range such as "from 1 to 6" should be considered to have specifically disclosed subranges such as "from 1 to 3", "from 1 to 4", "from 1 to 5", "from 2 to 4", "from 2 to 6", "from 3 to 6", etc.; as well as individual numbers within that range, for example, 1, 2, 3, 4, 5, and 6. This applies regardless of the breadth of the range.

Whenever a numerical range is indicated herein (for example "10-15", "10 to 15", or any pair of numbers linked by these another such range indication), it is meant to include any number (fractional or integral) within the indicated range limits, including the range limits, unless the context clearly dictates otherwise. The phrases "range/ranging/ranges between" a first indicate number and a second indicate number and "range/ranging/ranges from" a first indicate number "to", "up to", "until" or "through" (or another such range-indicating term) a second indicate number are used herein interchangeably and are meant to include the first and second indicated numbers and all the fractional and integral numbers therebetween.

Unless otherwise indicated, numbers used herein and any number ranges based thereon are approximations within the accuracy of reasonable measurement and rounding errors as understood by persons skilled in the art.

It is appreciated that certain features of the invention, which are, for clarity, described in the context of separate embodiments, may also be provided in combination in a single embodiment. Conversely, various features of the invention, which are, for brevity, described in the context of a single embodiment, may also be provided separately or in any suitable subcombination or as suitable in any other described embodiment of the invention. Certain features described in the context of various embodiments are not to be considered essential features of those embodiments, unless the embodiment is inoperative without those elements.

Although the invention has been described in conjunction with specific embodiments thereof, it is evident that many alternatives, modifications and variations will be apparent to those skilled in the art. Accordingly, it is intended to embrace all such alternatives, modifications and variations that fall within the scope of the appended claims.

## Claims

1. A biopsy assembly (**400**), comprising:
a biopsy tube (**404, 606, 710, 762, 738, 812**) defining a lumen (**406, 609, 708, 819**); a tissue penetrator (**402, 604, 702, 730, 750, 770, 800**) having a distal tip (**412, 612, 771, 808**) shaped to penetrate through tissue, wherein said tissue penetrator (**402, 604, 702, 730, 750, 770, 800**) is positioned within said lumen (**406, 609, 708, 819**) and comprising:
an elongated flexible body (**408, 608, 704, 721, 734, 776, 806**) shaped and sized to move within said biopsy tube lumen (**406, 609, 708, 819**);
a collapsible distal cutting portion (**410, 610, 706, 718, 732, 752, 772, 810**) coupled to said body (**408, 608, 704, 721, 734, 776, 806**) and extending out from said lumen (**406, 609, 708, 819**) in an expanded state in front of said biopsy tube (**404, 606, 710, 762, 738, 812**), and configured to collapse to a width smaller than an inner width of said lumen (**406, 609, 708, 819**) in a collapsed state,
wherein said collapsible distal cutting portion (410, 610, 706, 718, 732, 752, 772, 810) comprises a distal sharp cutting edge (411, 774, 775), configured to perform a thin cut through said tissue, wherein in an expanded state a maximal width of said collapsible distal cutting portion (410, 610, 706, 718, 732, 752, 772, 810) is larger than an inner width or an inner diameter of said biopsy tube lumen;
and **characterised in that** said collapsible distal cutting portion (**410, 610, 706, 718, 732, 752, 772, 810**) is coupled to a distal end of said body (**408, 608, 704, 721, 734, 776, 806**);
and said distal tip (**412, 612, 771, 808**) is an integral part of said collapsible distal cutting portion (**410, 610, 706, 718, 732, 752, 772, 810**).

2. A biopsy assembly according to claim 1, wherein said collapsible distal cutting portion (**410, 610, 706, 718, 732, 752, 772, 810**) is in a form of a thin and flexible layer.

3. A biopsy assembly according to any one of claims 1 or 2, wherein said collapsible distal cutting portion (**410, 610, 706, 718, 732, 752, 772, 810**) comprises said distal sharp cutting edge (**411, 774, 775**) on a tissue facing surface of said collapsible distal cutting portion (**410, 610, 706, 718, 732, 752, 772, 810**), configured to perform said thin cut through said tissue during the advancement of said tissue penetrator (**402, 604, 702, 730, 750, 770, 800**) into said tissue when said collapsible distal cutting portion (**410, 610, 706, 718, 732, 752, 772, 810**) extends from said biopsy tube (**404, 606, 710, 762, 738, 812**) and is in an expanded state, and wherein in said collapsed state said tissue penetrator (**402, 604, 702, 730, 750, 770, 800**) is configured to be retracted within said biopsy tube lumen (**406, 609, 708, 819**).

4. A biopsy assembly according to any one of the previous claims, wherein said collapsible distal cutting portion (**410, 610, 706, 718, 732, 752, 772, 810**) is integrated with said elongated flexible body (**408, 608, 704, 721, 734, 776, 806**).

5. A biopsy assembly according to any one of the previous claims, wherein said collapsible distal cutting portion (**410, 610, 706, 718, 732, 752, 772, 810**) is thin, having a ratio larger than 1:8 between thickness and maximal width or diameter of said collapsible distal cutting portion (**410, 610, 706, 718, 732, 752, 772, 810**).

6. A biopsy assembly according to any one of the previous claims, wherein a thickness of said distal sharp cutting edge (**411, 774, 775**) is smaller than 0.1 mm.

7. A biopsy assembly according to any one of the previous claims, wherein a tissue facing surface of said tissue penetrator (**402, 604, 702, 730, 750, 770, 800**) comprising said collapsible distal cutting portion (**410, 610, 706, 718, 732, 752, 772, 810**) and said distal sharp cutting edge (**411, 774, 775**) is at least partly angled or at least partly curved.

8. A biopsy assembly according to claim 5, wherein an angle between said tissue facing surface and said tissue is larger than 20 degrees.

9. A biopsy assembly according to any one of the previous claims, wherein a cross-section of a circumference of said collapsible distal cutting portion (**410, 610, 706, 718, 732, 752, 772, 810**), in an expanded state, is a continuous arc.

10. A biopsy assembly according to claim 9, wherein said arc subtends an angle of at least 30 degrees and/or has a radius of curvature in a range of 0.2-20 mm, and/or has a length in said expanded state of up to 30 mm.

11. A biopsy assembly according to any one of the previous claims, comprising:
a coupler (**504**) configured to reversibly couple said elongated flexible body (**408, 608, 704, 721, 734, 776, 806**) of said tissue penetrator (**402, 604, 702, 730, 750, 770, 800**) to said biopsy tube (**404, 606, 710, 762, 738, 812**).

12. A biopsy assembly according to any one of the previous claims, wherein said collapsible cutting portion (**410, 610, 706, 718, 732, 752, 772, 810**) is formed from a single thin and flexible layer of material configured to bend around said elongated flexible body (**408, 608,** 704, 721, 734, 776, 806) or to furl at least partly around said elongated flexible body (**408, 608, 704, 721, 734, 776, 806**), to a width smaller than an inner width of said lumen (**406, 609, 708, 819**).

13. A biopsy assembly according to any one of the previous claims, wherein said collapsible distal cutting portion (**410, 610, 706, 718, 732, 752, 772, 810**) is rigid in an axial direction and bendable in a lateral and/or a tangential direction.

14. A biopsy assembly according to any one of the previous claims, wherein a minimal thickness of a wall of said biopsy tube (**404, 606, 710, 762, 738, 812**) surrounding said lumen is at least 10% of the inner diameter of the biopsy tube (**404, 606, 710, 762, 738, 812**).

15. A biopsy assembly according to any one of the previous claims, wherein a minimal thickness of a wall of said biopsy tube (**404, 606, 710, 762, 738, 812**) is 0.05 mm.

## Patentansprüche

1. Biopsievorrichtung (**400**), umfassend:
eine Biopsieröhre (**404, 606, 710, 762, 738, 812**), die ein Lumen (**406, 609, 708, 819**) definiert;
einen Gewebepenetrator (**402, 604, 702, 730, 750, 770, 800**) mit einer distalen Spitze (**412, 612, 771, 808**), die geformt ist, um durch Gewebe zu penetrieren, wobei der Gewebepenetrator (**402, 604, 702, 730, 750, 770, 800**) innerhalb des Lumens (**406, 609, 708, 819**) positioniert ist und umfassend:
einen länglichen flexiblen Körper (**408, 608, 704, 721, 734, 776, 806**), der geformt und dimensioniert ist, um sich innerhalb des Biopsieröhrenlumens (**406, 609, 708, 819**) zu bewegen;
einen zusammenklappbaren distalen Schneidabschnitt (**410, 610, 706, 718, 732, 752, 772, 810**), der mit dem Körper (**408, 608, 704, 721, 734, 776, 806**) gekoppelt ist und in einem expandierten Zustand vor der Biopsieröhre (**404, 606, 710, 762, 738, 812**) aus dem Lumen (**406, 609, 708, 819**) herausragt, und konfiguriert ist, um in einem zusammengeklappten Zustand auf eine Breite zu kollabieren, die kleiner als eine Innenbreite des Lumens (**406, 609, 708, 819**) ist,
wobei der zusammenklappbare distale Schneidabschnitt (410, 610, 706, 718, 732, 752, 772, 810) eine distale scharfe Schneidkante (411, 774, 775) umfasst, die konfiguriert ist, um einen dünnen Schnitt durch das Gewebe durchzuführen, wobei in einem expandierten Zustand eine maximale Breite des zusammenklappbaren distalen Schneidabschnitts (410, 610, 706, 718, 732, 752, 810) größer als eine Innenbreite oder ein Innendurchmesser des Biopsieröhrenlumens ist; und **dadurch gekennzeichnet, dass**
der zusammenklappbare distale Schneidabschnitt (**410, 610, 706, 718, 732, 752, 772, 810**) mit einem distalen Ende des Körpers (**408, 608, 704, 721, 734, 776, 806**) gekoppelt ist; und
die distale Spitze (**412, 612, 771, 808**) ein integraler Bestandteil des zusammenklappbaren distalen Schneidabschnitts (**410, 610, 706, 718, 732, 752, 772, 810**) ist.

2. Biopsievorrichtung nach Anspruch 1, wobei der zusammenklappbare distale Schneidabschnitt (**410, 610, 706, 718, 732, 752, 772, 810**) in Form einer dünnen und flexiblen Schicht vorliegt.

3. Biopsievorrichtung nach einem der Ansprüche 1 bis 2, wobei der zusammenklappbare distale Schneidabschnitt (**410, 610, 706, 718, 732, 752, 772, 810**) die distale scharfe Schneidkante (**411, 774, 775**) auf einer gewebezugewandten Oberfläche des zusammenklappbaren distalen Schneidabschnitts (**410, 610, 706, 718, 732, 752, 772, 810**) umfasst, die konfiguriert ist, um den dünnen Schnitt durch das Gewebe während des Vorschubs des Gewebepenetrators (**402, 604, 702, 730, 750, 770, 800**) in das Gewebe durchzuführen, wenn der zusammenklappbare distale Schneidabschnitt (**410, 610, 706, 718, 732, 752, 772, 810**) aus der Biopsieröhre (**404, 606, 710, 762, 738, 812**) herausragt und in einem expandierten Zustand ist, und wobei in dem zusammengeklappten Zustand der Gewebepenetrator (**402, 604, 702, 730, 750, 770, 800**) konfiguriert ist, um innerhalb des Biopsieröhrenlumens (**406, 609, 708, 819**) zurückgezogen zu werden.

4. Biopsievorrichtung nach einem der vorhergehenden Ansprüche, wobei der zusammenklappbare distale Schneidabschnitt (**410, 610, 706, 718, 732, 752, 772, 810**) mit dem länglichen flexiblen Körper (**408, 608, 704, 721, 734, 776, 806**) integriert ist.

5. Biopsievorrichtung nach einem der vorhergehenden Ansprüche, wobei der zusammenklappbare distale Schneidabschnitt (**410, 610, 706, 718, 732, 752, 772, 810**) dünn ist und ein Verhältnis größer als 1:8 zwischen Dicke und maximaler Breite oder Durchmesser des zusammenklappbaren distalen Schneidabschnitts (**410, 610, 706, 718, 732, 752, 772, 810**) aufweist.

6. Biopsievorrichtung nach einem der vorhergehenden Ansprüche, wobei eine Dicke der distalen scharfen Schneidkante (**411, 774, 775**) kleiner als 0,1 mm ist.

7. Biopsievorrichtung nach einem der vorhergehenden Ansprüche, wobei eine gewebezugewandte Oberfläche des Gewebepenetrators (**402, 604, 702, 730, 750, 770, 800**), die den zusammenklappbaren distalen Schneidabschnitt (**410, 610, 706, 718, 732, 752, 772, 810**) und die distale scharfe Schneidkante (**411, 774, 775**) umfasst, zumindest teilweise abgewinkelt oder zumindest teilweise gekrümmt ist.

8. Biopsievorrichtung nach Anspruch 5, wobei ein Winkel zwischen der gewebezugewandten Oberfläche und dem Gewebe größer als 20 Grad ist.

9. Biopsievorrichtung nach einem der vorhergehenden Ansprüche, wobei ein Querschnitt eines Umfangs des zusammenklappbaren distalen Schneidabschnitts (**410, 610, 706, 718, 732, 752, 772, 810**) in einem expandierten Zustand ein kontinuierlicher Bogen ist.

10. Biopsievorrichtung nach Anspruch 9, wobei der Bogen einen Winkel von mindestens 30 Grad umspannt und/oder einen Krümmungsradius im Bereich von 0,2 bis 20 mm aufweist, und/oder eine Länge in dem expandierten Zustand von bis zu 30 mm aufweist.

11. Biopsievorrichtung nach einem der vorhergehenden Ansprüche, umfassend:
einen Koppler (504), der konfiguriert ist, um den länglichen flexiblen Körper (**408, 608, 704, 721, 734, 776, 806**) des Gewebepenetrators (**402, 604, 702, 730, 750, 770, 800**) reversibel mit der Biopsieröhre (**404, 606, 710, 762, 738, 812**) zu koppeln.

12. Biopsievorrichtung nach einem der vorhergehenden Ansprüche, wobei der zusammenklappbare Schneidabschnitt (**410, 610, 706, 718, 732, 752, 772, 810**) aus einer einzelnen dünnen und flexiblen Schicht aus Material gebildet ist, die konfiguriert ist, um sich um den länglichen flexiblen Körper (**408, 608, 704, 721, 734, 776, 806**) zu biegen oder sich zumindest teilweise um den länglichen flexiblen Körper (**408, 608, 704, 721, 734, 776, 806**) einzurollen, auf eine Breite, die kleiner als eine Innenbreite des Lumens (**406, 609, 708, 819**) ist.

13. Biopsievorrichtung nach einem der vorhergehenden Ansprüche, wobei der zusammenklappbare distale Schneidabschnitt (**410, 610, 706, 718, 732, 752, 772, 810**) in einer axialen Richtung starr und in einer lateralen und/oder einer tangentialen Richtung biegbar ist.

14. Biopsievorrichtung nach einem der vorhergehenden Ansprüche, wobei eine minimale Dicke einer Wand der Biopsieröhre (**404, 606, 710, 762, 738, 812**), die das Lumen umgibt, mindestens 10% des Innendurchmessers der Biopsieröhre (**404, 606, 710, 762, 738, 812**) beträgt.

15. Biopsievorrichtung nach einem der vorhergehenden Ansprüche, wobei eine minimale Dicke einer Wand der Biopsieröhre (**404, 606, 710, 762, 738, 812**) 0,05 mm beträgt.

## Revendications

1. Ensemble de biopsie (**400**), comprenant :
un tube de biopsie (**404, 606, 710, 762, 738, 812**) définissant une lumière (**406, 609, 708, 819**) ;
un pénétrateur de tissu (**402, 604, 702, 730, 750, 770, 800**) ayant une pointe distale (**412, 612, 771, 808**) formée pour pénétrer à travers le tissu, dans lequel ledit pénétrateur de tissu (**402, 604, 702, 730, 750, 770, 800**) est positionné à l'intérieur de ladite lumière (**406, 609, 708, 819**) et comprenant:
un corps flexible allongé (**408, 608, 704, 721, 734, 776, 806**) formé et dimensionné pour se déplacer à l'intérieur de ladite lumière du tube de biopsie (**406, 609, 708, 819**) ;
une partie de coupe distale rétractable (**410, 610, 706, 718, 732, 752, 772, 810**) couplée audit corps **(408, 608, 704, 721, 734, 776, 806)** et s'étendant hors de ladite lumière (**406, 609, 708, 819**) dans un état déployé devant ledit tube de biopsie (**404, 606, 710, 762, 738, 812**), et configurée pour se rétracter à une largeur plus petite qu'une largeur interne de ladite lumière (**406, 609, 708, 819**) dans un état rétracté,
dans lequel ladite partie de coupe distale rétractable (410, 610, 706, 718, 732, 752, 772, 810) comprend un bord de coupe distal tranchant (411, 774, 775), configuré pour effectuer une coupe fine à travers ledit tissu, dans lequel dans un état déployé une largeur maximale de ladite partie de coupe distale rétractable (410, 610, 706, 718, 732, 752, 810) est plus grande qu'une largeur interne ou qu'un diamètre interne de ladite lumière du tube de biopsie ; et **caractérisé en ce que**
ladite partie de coupe distale rétractable (**410, 610, 706, 718, 732, 752, 772, 810**) est couplée à une extrémité distale dudit corps (**408, 608, 704, 721, 734, 776, 806**) ; et
ladite pointe distale (**412, 612, 771, 808**) est une partie intégrante de ladite partie de coupe distale rétractable (**410, 610, 706, 718, 732, 752, 772, 810**).

2. Ensemble de biopsie selon la revendication 1, dans lequel ladite partie de coupe distale rétractable (**410, 610, 706, 718, 732, 752, 772, 810**) est sous forme d'une couche mince et flexible.

3. Ensemble de biopsie selon l'une quelconque des revendications 1 à 2, dans lequel ladite partie de coupe distale rétractable (**410, 610, 706, 718, 732, 752, 772, 810**) comprend ledit bord de coupe distal tranchant (**411, 774, 775**) sur une surface faisant face au tissu de ladite partie de coupe distale rétractable (**410, 610, 706, 718, 732, 752, 772, 810**), configurée pour effectuer ladite coupe fine à travers ledit tissu pendant l'avancement dudit pénétrateur de tissu (**402, 604, 702, 730, 750, 770, 800**) dans ledit tissu lorsque ladite partie de coupe distale rétractable (**410, 610, 706, 718, 732, 752, 772, 810**) s'étend depuis ledit tube de biopsie (**404, 606, 710, 762, 738, 812)** et est dans un état déployé, et dans lequel dans ledit état rétracté ledit pénétrateur de tissu (**402, 604, 702, 730, 750, 770, 800**) est configuré pour être rétracté à l'intérieur de ladite lumière du tube de biopsie (**406, 609, 708, 819**).

4. Ensemble de biopsie selon l'une quelconque des revendications précédentes, dans lequel ladite partie de coupe distale rétractable (**410, 610, 706, 718, 732, 752, 772, 810**) est intégrée avec ledit corps flexible allongé (**408, 608, 704, 721, 734, 776, 806**).

5. Ensemble de biopsie selon l'une quelconque des revendications précédentes, dans lequel ladite partie de coupe distale rétractable (**410, 610, 706, 718, 732, 752, 772, 810**) est mince, ayant un rapport supérieur à 1:8 entre l'épaisseur et la largeur maximale ou le diamètre de ladite partie de coupe distale rétractable (**410, 610, 706, 718, 732, 752, 772, 810**).

6. Ensemble de biopsie selon l'une quelconque des revendications précédentes, dans lequel une épaisseur dudit bord de coupe distal tranchant (**411, 774, 775**) est inférieure à 0,1 mm.

7. Ensemble de biopsie selon l'une quelconque des revendications précédentes, dans lequel une surface faisant face au tissu dudit pénétrateur de tissu (**402, 604, 702, 730, 750, 770, 800**) comprenant ladite partie de coupe distale rétractable (**410, 610, 706, 718, 732, 752, 772, 810**) et ledit bord de coupe distal tranchant (**411, 774, 775**) est au moins partiellement inclinée ou au moins partiellement courbée.

8. Ensemble de biopsie selon la revendication 5, dans lequel un angle entre ladite surface faisant face au tissu et ledit tissu est supérieur à 20 degrés.

9. Ensemble de biopsie selon l'une quelconque des revendications précédentes, dans lequel une section transversale d'une circonférence de ladite partie de coupe distale rétractable (**410, 610, 706, 718, 732, 752, 772, 810**), dans un état déployé, est un arc continu.

10. Ensemble de biopsie selon la revendication 9, dans lequel ledit arc sous-tend un angle d'au moins 30 degrés et/ou a un rayon de courbure dans une plage de 0,2 à 20 mm, et/ou a une longueur dans ledit état déployé allant jusqu'à 30 mm.

11. Ensemble de biopsie selon l'une quelconque des revendications précédentes, comprenant :
un coupleur (**504**) configuré pour coupler de façon réversible ledit corps flexible allongé (**408, 608, 704, 721, 734, 776, 806**) dudit pénétrateur de tissu (**402, 604, 702, 730, 750, 770, 800**) audit tube de biopsie (**404, 606, 710, 762, 738, 812**).

12. Ensemble de biopsie selon l'une quelconque des revendications précédentes, dans lequel ladite partie de coupe rétractable (**410, 610, 706, 718, 732, 752, 772, 810**) est formée à partir d'une seule couche mince et flexible de matériau configurée pour se plier autour dudit corps flexible allongé (**408, 608, 704, 721, 734, 776, 806**) ou pour s'enrouler au moins partiellement autour dudit corps flexible allongé (**408, 608, 704, 721, 734, 776, 806**), à une largeur plus petite qu'une largeur interne de ladite lumière (**406, 609, 708, 819**).

13. Ensemble de biopsie selon l'une quelconque des revendications précédentes, dans lequel ladite partie de coupe distale rétractable (**410, 610, 706, 718, 732, 752, 772, 810**) est rigide dans une direction axiale et pliable dans une direction latérale et/ou une direction tangentielle.

14. Ensemble de biopsie selon l'une quelconque des revendications précédentes, dans lequel une épaisseur minimale d'une paroi dudit tube de biopsie (**404, 606, 710, 762, 738, 812**) entourant ladite lumière est au moins 10% du diamètre interne du tube de biopsie (**404, 606, 710, 762, 738, 812**).

15. Ensemble de biopsie selon l'une quelconque des revendications précédentes, dans lequel une épaisseur minimale d'une paroi dudit tube de biopsie (**404, 606, 710, 762, 738, 812**) est de 0,05 mm.
